# EUROPEAN PATENT SPECIFICATION

(11) **EP 3 986 292 B1**
(45) Date of publication and mention of the grant of the patent: **17.04.2024**
(21) Application number: 20733115.8
(22) Date of filing: 27.05.2020
(51) Int. Cl.: A61B 17/22, A61B 18/26, A61B 17/00, A61B 18/00, A61B 18/22, A61B 18/24

(54) **BALLOON SURFACE PHOTOACOUSTIC PRESSURE WAVE GENERATION TO DISRUPT VASCULAR LESIONS**
FOTOAKUSTISCHE DRUCKWELLENERZEUGUNG AN DER BALLONOBERFLÄCHE ZUR ZERSTÖRUNG VON GEFÄSSVERLETZUNGEN
GÉNÉRATION D'ONDES DE PRESSION PHOTOACOUSTIQUES DEPUIS UNE SURFACE DE BALLONNET POUR RÉDUIRE DES LÉSIONS VASCULAIRES

(30) Priority: 19.06.2019 US 201962863506 P
(43) Date of publication of application: 27.04.2022
(73) Proprietor: Boston Scientific Scimed, Inc., Maple Grove, MN 55311 (US)
(72) Inventor: MCGOWAN, Roger, W., Otsego, MN 55362 (US); MASSIMINI, Daniel, Frank, Brooklyn Park, MN 55443 (US); SHAO, Haiping, Plymouth, MN 55446 (US); SMUK, Christopher, Champlin, MN 55316 (US)
(74) Representative: Vossius & Partner Patentanwälte Rechtsanwälte mbB
(86) International application number: PCT/US2020/034642
(87) International publication number: WO 2020/256898

(56) References cited:
- WO-A1-2014/022867
- US-A- 5 944 687
- US-A1- 2006 270 976
- US-A1- 2016 184 023

## Description

### RELATED APPLICATIONS

This application claims priority on United States Provisional Application Serial No. 62/863,506, filed on June 19, 2019, and entitled "BALLOON SURFACE PHOTOACOUSTIC SHOCKWAVE GENERATION TO DISRUPT VASCULAR LESIONS", and on United States Patent Application Serial No. 16/884,257 filed on May 27, 2020, and entitled "BALLOON SURFACE PHOTOACOUSTIC SHOCKWAVE GENERATION TO DISRUPT VASCULAR LESIONS".

### BACKGROUND

Vascular lesions within blood vessels in the body can be associated with an increased risk for major adverse events, such as myocardial infarction, embolism, deep vein thrombosis, stroke, and the like. Severe vascular lesions can be difficult to treat and achieve patency for a physician in a clinical setting.

Vascular lesions may be treated using interventions such as drug therapy, balloon angioplasty, atherectomy, stent placement, vascular graft bypass, to name a few. Such interventions may not always be ideal or may require subsequent treatment to address the lesion.

US 2016/184023 discloses devices and methods for using laser-induced pressure waves to disrupt vascular blockages.

### SUMMARY

The photoacoustic catheter of the invention is defined in claim 1. Embodiments are defined in the dependent claims. In a first aspect, the present disclosure further provides a photoacoustic catheter adapted for placement within a blood vessel having a vessel wall is provided. The photoacoustic catheter can have an elongate shaft extending from a proximal region to a distal region. The elongate shaft can include a light guide, where the light guide can be configured to be placed in optical communication with a light source. The photoacoustic catheter can include a balloon coupled to the elongate shaft, where the balloon can be configured to expand from a collapsed configuration suitable for advancing the photoacoustic catheter through a patient's vasculature to a first expanded configuration suitable for anchoring the photoacoustic catheter in position relative to a treatment site. The photoacoustic catheter can include a photoacoustic transducer disposed on a surface of the balloon and in optical communication with the light guide. The photoacoustic transducer can include a light-absorbing material and a thermal expansion material.

In a second aspect, in addition to one or more of the preceding or following aspects, or in the alternative to some aspects, the present disclosure further provides a photoacoustic catheter in which the photoacoustic transducer is located on an outer surface of the balloon.

In a third aspect of the disclosure and according to the invention, the photoacoustic transducer is located on an inner surface of the balloon.

In a fourth aspect of the present disclosure, in addition to one or more of the preceding or following aspects, or in the alternative to some aspects, the photoacoustic transducer includes a conformal coating on the surface of the balloon extending continuously from a proximal location to distal location and extending continuously around a circumference of the balloon.

In a fifth aspect of the present disclosure, in addition to one or more of the preceding or following aspects, or in the alternative to some aspects, the photoacoustic transducer can be configured as a plurality of circumferential bars, longitudinal bars, diagonal bars, or islands.

In a sixth aspect of the present disclosure, in addition to one or more of the preceding or following aspects, or in the alternative to some aspects, a distal portion of the light guide includes a diffraction grating pattern configured to, when the balloon is in the first expanded configuration, direct light from the light guide to one or more light pattern locations.

In a seventh aspect of the present disclosure, in addition to one or more of the preceding or following aspects, or in the alternative to some aspects, a wall of the balloon includes integrated fluid bubbles.

In an eighth aspect of the present disclosure, in addition to one or more of the preceding or following aspects, or in the alternative to some aspects, the balloon can be configured to change from a first expanded configuration to a second, further expanded configuration.

In a ninth aspect of the present disclosure, in addition to one or more of the preceding or following aspects, or in the alternative to some aspects, the balloon includes a stress concentration structure and the photoacoustic transducer is located on a surface of the stress concentration structure.

In a tenth aspect of the present disclosure, in addition to one or more of the preceding or following aspects, or in the alternative to some aspects, the stress concentration structure includes a larger diameter region compared to a remainder of an adjacent balloon wall portion, includes a dome structure, or includes a rectangular structure.

In an eleventh aspect of the present disclosure, in addition to one or more of the preceding or following aspects, or in the alternative to some aspects, the thermal expansion material of the photoacoustic transducer is a polymer, and where the polymer is in thermal contact with the light absorbing material.

In a twelfth aspect of the present disclosure, in addition to one or more of the preceding or following aspects, or in the alternative to some aspects, the thermal expansion material is selected from a group including polydimethylsiloxane (PDMS), polytetrafluoroethylene (PTFE), polyimide, polyisobutylene (PIB), PIB polyurethane, polyurethanes, styrene isoprene butadiene, ethylene propylene polyacrylic, ethylene acrylic, fluorosilicone, polybutadiene, polyisoprene, and thermoplastic elastomers.

In a thirteenth aspect of the present disclosure, in addition to one or more of the preceding or following aspects, or in the alternative to some aspects, the light-absorbing material is selected from a group including nanoparticles, carbon nanotubes, candle soot, candle soot nanoparticles, carbon black, a nanotube array, multiwall carbon nanotubes, and light absorbing dye.

In a fourteenth aspect of the present disclosure, in addition to one or more of the preceding or following aspects, or in the alternative to some aspects, the light guide is an optical fiber and the light source is a laser.

In a fifteenth aspect of the present disclosure, in addition to one or more of the preceding or following aspects, or in the alternative to some aspects, and according to the invention, the photoacoustic catheters herein can include an outer balloon surrounding the balloon having the photoacoustic transducer.

In a sixteenth aspect of the present disclosure and according to the invention, a photoacoustic catheter adapted for placement within a blood vessel having a vessel wall is provided. The photoacoustic catheter includes an elongate shaft extending from a proximal region to a distal region, where the elongate shaft includes a light guide. The light guide is configured to be placed in optical communication with a light source. The photoacoustic catheter includes an outer balloon coupled to the elongate shaft, where the outer balloon is configured to expand from a collapsed configuration suitable for advancing the photoacoustic catheter through a patient's vasculature to a first expanded configuration suitable for anchoring the photoacoustic catheter in position relative to a treatment site. The photoacoustic catheter further includes an inner balloon coupled to the elongate shaft within the outer balloon, the inner balloon configured to expand from a collapsed configuration suitable for advancing the photoacoustic catheter through a patient's vasculature to a first expanded configuration. The inner balloon includes a photoacoustic transducer disposed on a surface of the inner balloon and in optical communication with the light guide. The photoacoustic transducer includes a light-absorbing material and a thermal expansion material. The photoacoustic catheter is configured to expand the outer balloon using an outer balloon inflation fluid and to expand the inner balloon using an inner balloon inflation fluid.

In a seventeenth aspect of the present disclosure, in addition to one or more of the preceding or following aspects, or in the alternative to some aspects, the outer balloon inflation fluid can be a liquid and the inner balloon inflation fluid can be a gas.

In an eighteenth aspect of the present disclosure, in addition to one or more of the preceding or following aspects, or in the alternative to some aspects, the inner balloon can be configured to include a concave portion when in the first expanded configuration, the photoacoustic transducer is located on an outer surface of the inner balloon, and the concave portion can be configured to focus at least one acoustic pressure wave from the photoacoustic transducer.

In a nineteenth aspect of the present disclosure, a method for photoacoustically generating pressure waves within a blood vessel is provided. Surgical methods are described with reference to the catheter of the present invention. Whilst no claim is directed to these methods per se, the methods are considered useful for the understanding of the invention. The method can include advancing a photoacoustic catheter to a treatment site within the blood vessel, where the photoacoustic catheter can include an elongate shaft, a balloon coupled to the elongate shaft, a light guide, and a photoacoustic transducer disposed on a surface of the balloon. The method can include expanding the balloon from a collapsed configuration suitable for advancing the photoacoustic catheter through a patient's vasculature to a first expanded configuration suitable for anchoring the photoacoustic catheter in position relative to the treatment site. The method can include after expanding the balloon, activating a light source in optical communication with the light guide and the photoacoustic transducer, thereby imparting acoustic pressure waves upon the treatment site.

In a twentieth aspect of the present disclosure, in addition to one or more of the preceding or following aspects, or in the alternative to some aspects, the method can further include, after activating the light source, further expanding the balloon from the first expanded configuration to a second further expanded configuration.

In yet another aspect of the present disclosure, in addition to one or more of the preceding aspects, or in the alternative to some aspects, the photoacoustic catheter can include a thermal expansion material and a light-absorbing material that are positioned adjacent to one another in layers.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope of the invention is defined by the appended claims.

### BRIEF DESCRIPTION OF THE DRAWINGS

Aspects may be more completely understood in connection with the following figures (FIGS.), in which:
FIG. 1 is a schematic cross-sectional view of a photoacoustic catheter in accordance with various embodiments herein.
FIG. 2 is a schematic cross-sectional view of a photoacoustic catheter in accordance with various embodiments herein.
FIG. 3 is a schematic cross-sectional view of a photoacoustic catheter in accordance with various embodiments herein.
FIG. 4 is a schematic side-plan view of a photoacoustic catheter in accordance with various embodiments herein.
FIGS. 5-7 are schematic side-plan views of additional configurations of the photoacoustic catheter in accordance with various embodiments herein.
FIG. 8 is a schematic cross-sectional view of a photoacoustic catheter in accordance with various embodiments herein.
FIG. 9 is a schematic cross-sectional view of a photoacoustic catheter in accordance with various embodiments herein.
FIG. 10 is a schematic cross-sectional view of a photoacoustic catheter in accordance with various embodiments herein.
FIG. 11 is a schematic cross-sectional view of a photoacoustic catheter in accordance with various embodiments herein.
FIG. 12 is a schematic cross-sectional view of a photoacoustic catheter in accordance with various embodiments herein.
FIG. 13 is a schematic cross-sectional view of a photoacoustic catheter in accordance with various embodiments herein.
FIG. 14 is a schematic diagram of a light pattern formed by a diffraction grating in accordance with various embodiments herein.
FIGS. 15-17 are schematic diagrams of additional configurations of light patterns formed by diffraction gratings in accordance with various embodiments herein.
FIG. 18 is a schematic cross-sectional view of a light guide in accordance with various embodiments herein.
FIG. 19 is a schematic cross-sectional view of a light guide in accordance with various embodiments herein.
FIG. 20 is a schematic cross-sectional view of a photoacoustic catheter in accordance with an embodiment of the invention.
FIG. 21 is a schematic cross-sectional view of the photoacoustic catheter of FIG. 20 along line 21-21' in accordance with various embodiments herein.
FIG. 22 is a schematic cross-sectional view of an additional configuration of the photoacoustic catheter of FIG. 20 along line 21-21' in accordance with various embodiments herein.
FIG. 23 is a schematic flow diagram for a method in accordance with the various embodiments herein.
FIG. 24 is a schematic cross-sectional view of a portion of a photoacoustic catheter in accordance with various embodiments herein.
FIG. 25 is a schematic cross-sectional view of a portion of a photoacoustic catheter in accordance with various embodiments herein.
FIG. 26 is a schematic cross-sectional view of a portion of a photoacoustic catheter in accordance with various embodiments herein.
FIG. 27 is a schematic cross-sectional view of an elongate shaft and multiple light guides of a photoacoustic catheter along line 27-27' in FIG. 1 in accordance with various embodiments herein.
FIGS. 28-30 are schematic cross-sectional views of additional configurations for an elongate shaft and multiple light guides of a photoacoustic catheter along line 27-27' in FIG. 1 in accordance with various embodiments herein.
FIGS. 31 -42 are schematic cross-sectional views of additional embodiments of an elongate shaft of a photoacoustic catheter in accordance with various embodiments herein.
FIGS. 43-45 are schematic cross-sectional views of various embodiments of a distal portion of a light guide of a photoacoustic catheter in accordance with various embodiments herein.

While embodiments are susceptible to various modifications and alternative forms, specifics thereof have been shown by way of example and drawings, and will be described in detail. It should be understood, however, that the scope herein is not limited to the particular aspects described but is defined by the claims.

### DESCRIPTION

Treatment of vascular lesions can reduce major adverse events or death in affected subjects. A major adverse event is one that can occur anywhere within the body due to the presence of a vascular lesion. Major adverse events can include, but are not limited to major adverse cardiac events, major adverse events in the peripheral or central vasculature, major adverse events in the brain, major adverse events in the musculature, or major adverse events in any of the internal organs.

The systems and methods disclosed herein describe the use of pressure waves for intravascular calcification disruption. In various embodiments herein, the pressure wave generation is accomplished using a photoacoustic catheter adapted for placement within a blood vessel. The photoacoustic catheters include an elongate shaft extending from a proximal region to a distal region. The elongate shafts can include a light guide (also sometimes referred to herein as a "first light guide", "second light guide", etc.) configured to be placed in optical communication with a light source. The photoacoustic catheters can include a balloon coupled to the elongate shaft and can be configured to expand from a collapsed configuration suitable for advancing the photoacoustic catheter through a patient's vasculature
to a first expanded configuration suitable for anchoring the photoacoustic catheter in position relative to a treatment site. The balloon includes a photoacoustic transducer disposed on a surface of the balloon and in optical communication with the light guide, where the photoacoustic transducer includes a light-absorbing material and a thermal expansion material.

As used herein, the terms "pressure wave," "acoustic wave," "acoustic pressure wave," or "sound wave" can be used interchangeably, and describe propagating a pressure disturbance in gaseous, liquid, or solid material medium, including vibrational waves, sound waves, ultrasonic waves and acoustic shock waves.

As used herein, the terms "intravascular lesion" or "vascular lesion", can be used interchangeably, and describe any lesion region within or adjacent to a vessel wall.

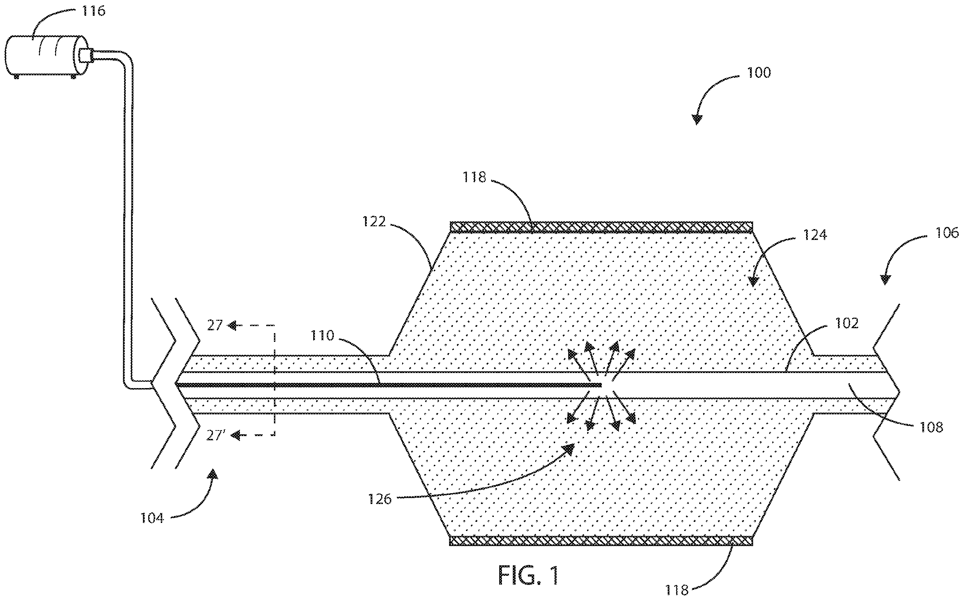

It will be appreciated that the photoacoustic catheters herein can include many different forms. Referring now to FIG. 1, a schematic cross-sectional view of a photoacoustic catheter 100 is shown in accordance with various embodiments herein. Photoacoustic catheter 100 can be adapted for placement within a blood vessel having a vessel wall. The photoacoustic catheter 100 can be used to treat a vascular lesion found within or adjacent to the vessel wall. In some embodiments, the vascular lesion can include a calcified vascular lesion. In some embodiments, the vascular lesion can include a fibrous vascular lesion. The photoacoustic catheter 100 can include an elongate shaft 102 extending from a proximal region 104 to a distal region 106, and can also include a lumen 108. In some embodiments, the photoacoustic catheter 100 can have a distal region opening and can accommodate and be tracked over a guide wire to a treatment location. In some embodiments, the photoacoustic catheter 100 does not include a lumen. In embodiments where the elongate shaft 102 does not include a lumen to be accessed by a caregiver, the elongate shaft 102 can be configured to allow the catheter to be steered through a patient's vasculature.

The elongate shaft 102 of photoacoustic catheter 100 can be coupled to a (also sometimes referred to herein simply as a "light guide") 110 in optical communication with a light source 116. In some embodiments, the first light guide 110 can be an optical fiber and the light source can be a laser. The light source 116 can be in optical communication with the first light guide 110 at a proximal region 104 of the elongate shaft 102. A schematic depiction of exemplary emitted light 126 as transmitted by the first light guide 110 is shown. It will be appreciated that, photoacoustic catheter 100 can include more than one light guide. In some embodiments, photoacoustic catheter 100 can include a second light guide, a third light guide, a fourth light guide, a fifth light guide, a sixth light guide, or more. In some embodiments, a plurality of light guides will be evenly spaced and radially offset from each other so that where there are *n* light guides, they are spaced apart by 360 degrees divided by *n.* In other embodiments, the light guides will be unevenly spaced and radially offset from each other.

It will be appreciated that the photoacoustic catheters herein can include any number of light guides. For example, in some embodiments, the photoacoustic catheters herein can include from one light guide to five light guides. In other embodiments, the photoacoustic catheters herein can include from five to fifteen light guides. In yet other embodiments, the photoacoustic catheters herein can include from ten light guides to thirty light guides. The photoacoustic catheters herein can include one, two, three, four, five, six, seven, eight, nine, or ten light guides. The photoacoustic catheters can include 11, 12, 13, 14, 15, 16, 17, 18, 19, 20, 21, 22, 23, 24, 25, 26, 27, 28, 29, or 30 light guides. It will be appreciated that photoacoustic catheters herein can include any number of light guides that can fall within a range, wherein any of the forgoing numbers can serve as the lower or upper bound of the range, provided that the lower bound of the range is a value less than the upper bound of the range. In some embodiments, the photoacoustic catheters herein can include more than 30 light guides.

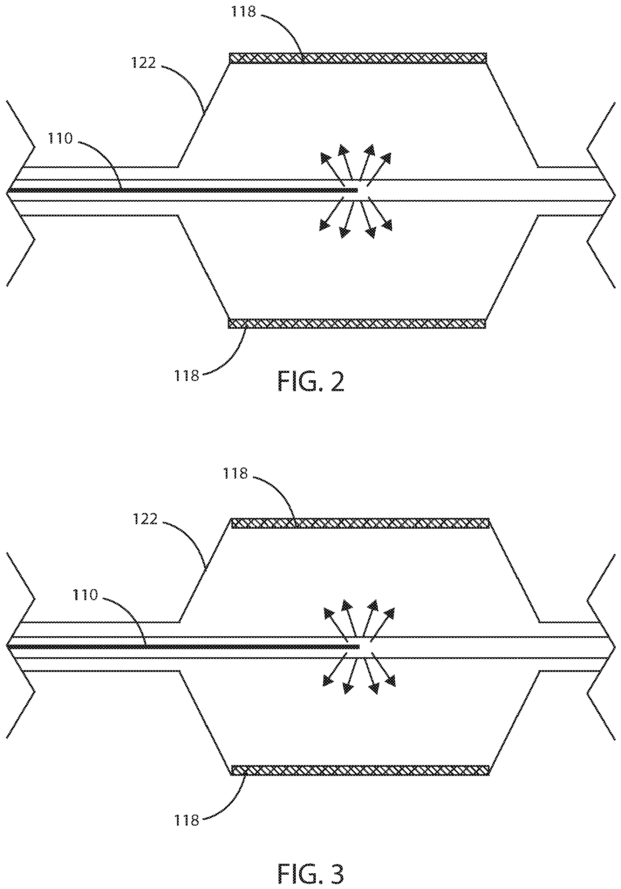

The photoacoustic catheter 100 includes a balloon 122. The balloon 122 can expand from a collapsed configuration suitable for advancing the catheter through a patient's vasculature to a first expanded configuration suitable for anchoring the catheter in position relative to a treatment site. Expansion of the balloons herein to various expanded configurations will be discussed in more detail below. The balloon 122 includes a photoacoustic transducer 118 disposed on a surface of the balloon 122 and in optical communication with the first light guide 110 to direct light 126 to the interior of balloon 122. In some embodiments, the photoacoustic transducer 118 is disposed on an outer surface of the balloon 122, as shown in FIG. 2. In other embodiments, the photoacoustic transducer 118 is disposed on an inner surface of the balloon 122, as shown in FIG. 3. The balloon 122 can be an inflatable balloon. In some embodiments, photoacoustic catheter 100 also includes an outer balloon that can surround balloon 122, where the photoacoustic transducer is disposed on balloon 122. In some embodiments, the balloon 122 is configured to change from a first expanded configuration to a second, further expanded configuration. It will be appreciated that the balloons herein can assume multiple expanded configurations between a first expanded configuration and a second further expanded configuration. In some embodiments, the balloons herein will include a maximum expanded configuration.

The photoacoustic transducer 118 can be adapted to impart acoustic pressure waves upon a calcified lesion to induce fractures in the calcified lesion. The photoacoustic transducer 118 can be formed from a light-absorbing material and a thermal expansion material. In some embodiments, the thermal expansion material of the photoacoustic transducer 118 is a polymer, and the polymer is in thermal contact with the light absorbing material. The thermal expansion material and the light absorbing material will both be discussed in more detail below. The balloon 122 can be inflated by introduction of a balloon inflation fluid 124. The balloon inflation fluid 124 can include a fluid suitable for transmitting acoustic energy from the first light guide 110 to an inside surface of the balloon 122. Exemplary balloon inflation fluids can include, but are not to be limited to one or more of water, saline, contrast medium, gases such as carbon dioxide, and the like. The balloon inflation fluids suitable for use herein can be tailored on the basis of composition, viscosity, density, and the like in order to manipulate the rate of travel of the acoustic pressure waves therein.

The photoacoustic transducer can be conformal and continuous on at least a portion of a surface of the balloon. In some embodiments, the photoacoustic transducer includes a conformal coating on a surface of the balloon 122 extending continuously from a proximal location to distal location and extending continuously around a circumference of the balloon 122, as shown in FIGS. 1-3.

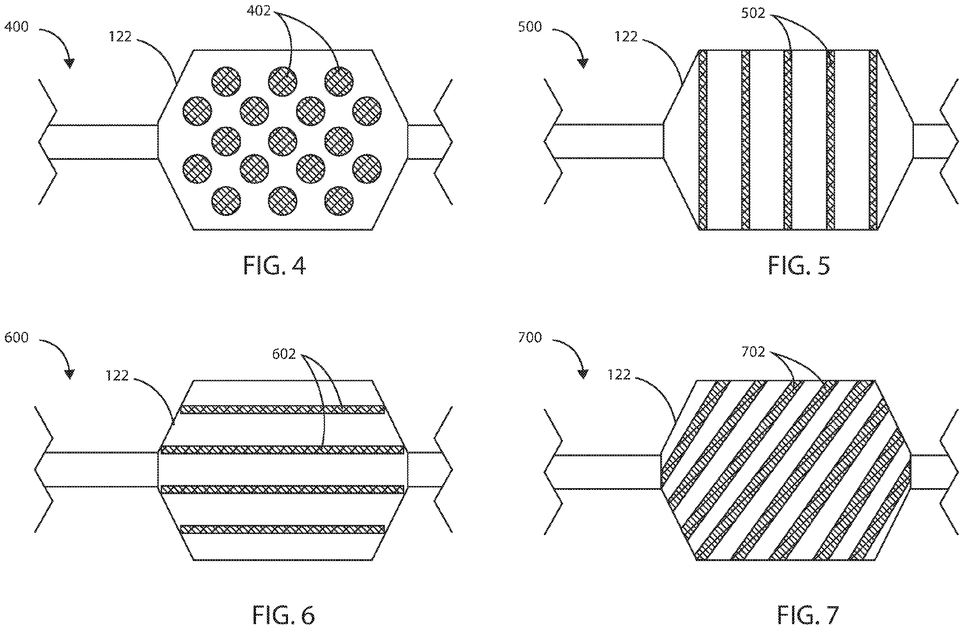

Referring now to FIGS. 4-7, schematic side-plan views of exemplary conformal and non-continuous photoacoustic transducer patterns are shown in accordance with various embodiments herein. The configuration of photoacoustic catheter 400 in FIG. 4 includes a photoacoustic transducer pattern having a plurality of islands 402 of the photoacoustic transducer disposed on a surface of the balloon 122. The islands can include, but are not to be limited to shapes including circles, ovals, diamonds, squares, rectangles, triangles, stars, symbols, letters, logos, and the like. The configuration of photoacoustic catheter 500 in FIG. 5 includes a photoacoustic transducer pattern of circumferential bars 502. The configuration of photoacoustic catheter 600 in FIG. 6 includes a photoacoustic transducer pattern of longitudinal bars 602. The configuration of photoacoustic catheter 700 in FIG. 7 includes a photoacoustic transducer pattern of diagonal bars 702. It will be appreciated that the continuous and non-continuous photoacoustic transducers described herein can be disposed on an external surface of the balloon or on an internal surface of the balloon. The conformal and non-continuous photoacoustic transducer examples of FIGS. 4-7 can be referred to as each including a single photoacoustic transducer that includes separate portions or as including a plurality of photoacoustic transducers, such as a first photoacoustic transducer and one or more of a second photoacoustic transducer, a third photoacoustic transducer, a fourth photoacoustic transducer, a fifth photoacoustic transducer, a sixth photoacoustic transducer, a seventh photoacoustic transducer, an eighth photoacoustic transducer, a ninth photoacoustic transducer, a tenth photoacoustic transducer, etc.

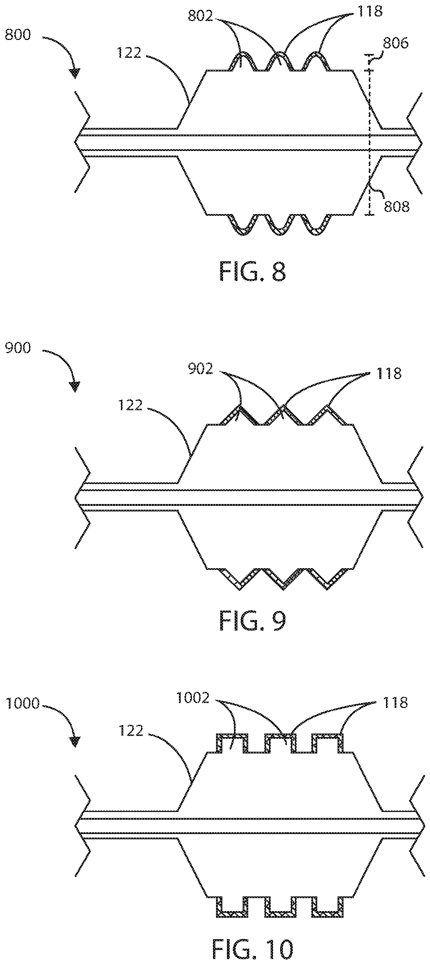

The photoacoustic transducer can be localized on various structures on a surface of the balloon 122. Referring now to FIGS. 8-10, schematic cross-sectional views of balloons with stress concentration structures disposed about the circumference are shown in accordance with various embodiments herein. The stress concentration structures can include a variety of shapes and sizes. The stress concentration structures can be made from a metal, a polymeric material, and the like. In some embodiments, the stress concentration structures can be integral to the balloon material. In some embodiments, the stress concentration structures can be attached to the balloon material.

The configuration of photoacoustic catheter 800 in FIG. 8 includes a stress concentration structure having a dome-shaped structure 802 in cross section. The configuration of photoacoustic catheter 900 in FIG. 9 includes a stress concentration structure having a triangle-shaped structure 902 (FIG. 9) in cross section. The configuration of photoacoustic catheter 1000 in FIG. 10 includes a stress concentration structure having a rectangular-shaped structure 1002 (FIG. 10) in cross section. The stress concentration structure can be integral to the balloon or can be physically distinct from the balloon. The stress concentration structure can be made from the same material as the balloon or can be made from a different material than the balloon. In the case of a physically distinct stress concentration structure, the stress concentration structure can be affixed to the surface of the balloon. In various embodiments, the stress concentration structures can be made using a contiguous balloon such that the balloon material can have different properties at different locations to form the stress concentration structures. For example, the balloon material can be more concentrated or of a different composition at different locations about the balloon to form the stress concentrations structures. By way of example, the stress concentration structures 802 of FIG. 8 can be formed integral with the balloon at different locations along or about the balloon.

In some examples, photoacoustic catheters having stress concentration structures include a photoacoustic transducer located on a surface of the stress concentration structure, such as an inner surface of the stress concentration structure or an outer surface of the stress concentration structure, which are shown in FIGS. 8-10. Referring now to FIG. 8, a schematic cross-sectional view of a photoacoustic catheter 800 is shown in accordance with various embodiments herein. The configuration of photoacoustic catheter 800 in FIG. 8 includes a stress concentration structure having a dome-shaped structure 802 on the outer surface of balloon 122 with a photoacoustic transducer 118 disposed on an outer surface of the stress concentration structure. The configuration of photoacoustic catheter 900 in FIG. 9 includes a stress concentration structure having a triangle-shaped structure 902 on the outer surface of balloon 122 with a photoacoustic transducer 118 disposed on an outer surface of the stress concentration structure. The configuration of photoacoustic catheter 1000 in FIG. 10 includes a stress concentration structure having a rectangular-shaped structure 1002 on the outer surface of balloon 122 with a photoacoustic transducer 118 disposed on an outer surface of the stress concentration structure.

It will be appreciated that the stress concentration structures include those having a larger diameter region compared to a remainder of an adjacent balloon wall portion. Referring to FIG. 8, the stress concentration structure can include those that have a height 806 extending in a direction perpendicular to an outer surface of the balloon, where balloon 122 has a diameter 808. Various diameters suitable for use with the balloons herein are described below. The stress concentration structures shown in FIGS. 9 and 10 also include a height extending in a direction perpendicular to an outer surface of the balloon, such that the stress concentration structures have a larger diameter region compared to a remainder of an adjacent balloon wall portion, as discussed in reference to FIG. 8. While not shown in FIGS. 8-10, it will be appreciated that the balloons herein can further include those having stress concentration structures on the outside of the balloon or incorporated into the balloon itself, and a photoacoustic transducer disposed on an inner surface of the balloon. By way of example, the balloon 122 of FIG. 3 could include stress concentration structures disposed on the outer surface of balloon 122 and the photoacoustic transducer 118 disposed on the inner surface. In some embodiments, the photoacoustic transducer disposed on an inner surface of the balloon 122 can conform to the shape of the stress concentration structures on the outer surface.

The stress concentration structures herein can include those that have a height 806 extending in a direction perpendicular to an outer surface of the balloon of from 1 micrometer (µm) to 500 µm. In some embodiments, the stress concentration structure can include those that have a height 806 extending in a direction perpendicular to an outer surface of the balloon of from 75 µm to 200 µm. In some embodiments, the stress concentration structure can include those that have a height 806 extending in a direction perpendicular to an outer surface of the balloon of from 1 µm to 50 µm. In some embodiments, the height 806 of the stress concentrator can be greater than or equal to 1 µm, 2 µm, 3 µm, 4 µm, 5 µm, 6 µm, 7 µm, 8 µm, 9 µm, 10 µm, 20 µm, 30 µm, 40 µm, 50 µm, 60 µm, 70 µm, 80 µm, 90 µm, 100 µm, 110 µm, 120 µm, 130 µm, 140 µm, 150 µm, 160 µm, 170 µm, 180 µm, 190 µm, 200 µm, 210 µm, 220 µm, 230 µm, 240 µm, 250 µm, 260 µm, 270 µm, 280 µm, 290 µm, 300 µm, 310 µm, 320 µm, 330 µm, 340 µm, 350 µm, 360 µm, 370 µm, 380 µm, 390 µm, 400 µm, 410 µm, 420 µm, 430 µm, 440 µm, 450 µm, 460 µm, 470 µm, 480 µm, 490 µm, or 500 µm, or can be an amount falling within a range between any of the foregoing.

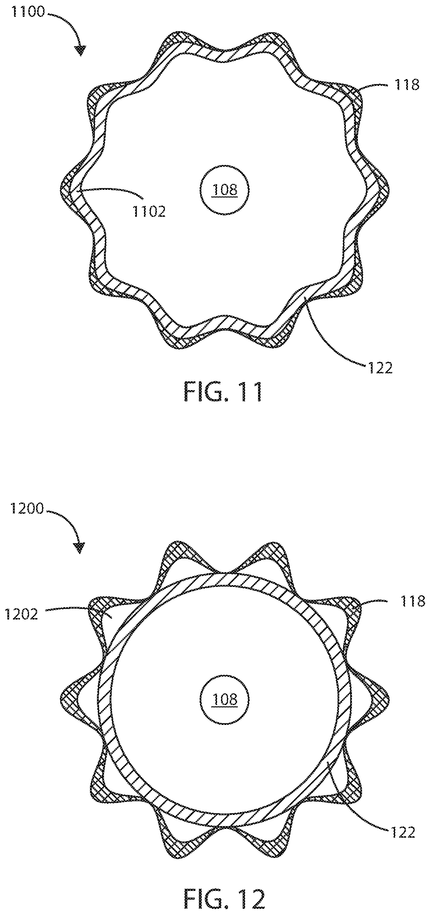

The photoacoustic transducer can also be localized along the longitudinal axis of balloon 122. Referring now to FIGS. 11-12, schematic cross-sectional views of balloons with a stress concentration structures disposed along the longitudinal axis are shown in accordance with various embodiments herein. The configuration of photoacoustic catheter 1100 in FIG. 11 includes balloon 122 surrounding a lumen 108 and stress concentration structures 1102 integrated into the balloon 122. A photoacoustic transducer 118 is disposed on the outer surface of the stress concentration structures 1102. The balloon 122 of photoacoustic catheter 1100 includes a variable inner diameter owing to the presence of the stress concentration structures 1102 that are integrated with the balloon 122. The configuration of photoacoustic catheter 1200 in FIG. 12 includes balloon 122 surrounding a lumen 108 and stress concentration structures 1202 affixed to the exterior of the balloon 122. A photoacoustic transducer 118 is disposed on the outer surface of the stress concentration structures 1202. The balloon 122 of 1200 includes a uniform inner diameter.

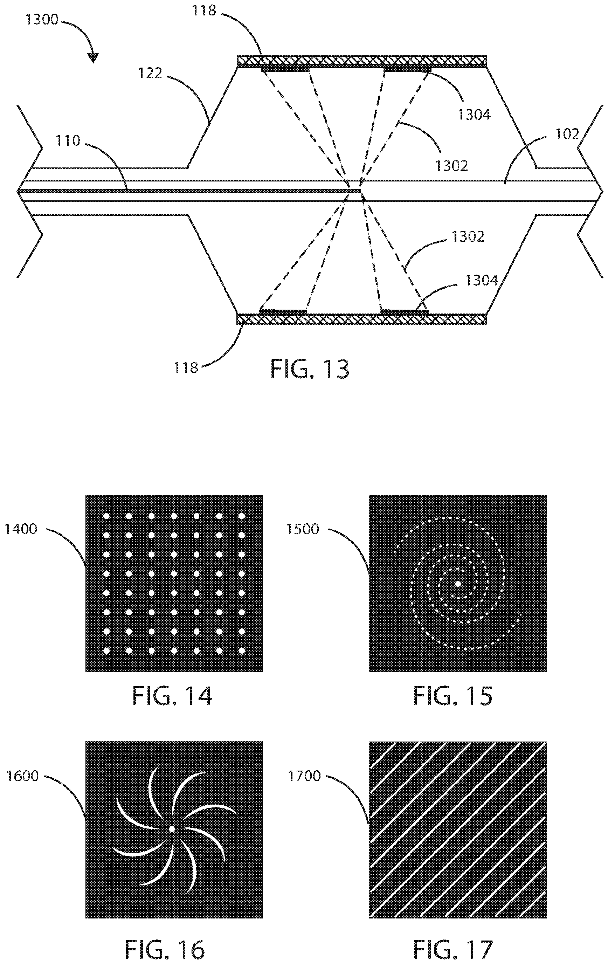

The photoacoustic catheters herein may include one or more diffraction gratings used to direct light from a light guide onto one or more specific locations on a surface of the balloon. Referring now to FIG. 13, a schematic cross-sectional view of a photoacoustic catheter 1300 is shown in accordance with various embodiments herein. The photoacoustic catheter 1300 includes a photoacoustic transducer 118 disposed about an outer surface of the balloon 122, where the balloon 122 can expand from a collapsed position to a first expanded configuration. The photoacoustic catheter 1300 includes an elongate shaft 102 and a first light guide 110 disposed along the elongate shaft 102. The first light guide 110 includes one or more diffraction grating patterns disposed along its length at a distal portion for directing or concentrating light 1302 from the first light guide 110 to one or more light pattern locations 1304 when the balloon 122 is in a first expanded configuration. The light pattern locations 1304 can include locations on balloon 122 or locations on the photoacoustic transducer 118. The light patterns formed by the diffraction gratings can selectively excite specific locations of the photoacoustic transducer 118 on a surface of the balloon.

The diffraction grating patterns can include, but are not to be limited to, the diffraction grating patterns shown in FIGS. 14-17. The configuration of diffraction grating patterns 1400 in FIG. 14 includes a dot matrix pattern. The configuration of diffraction grating patterns 1500 in FIG. 15 includes a spiral pattern. The configuration of diffraction grating patterns 1600 in FIG. 16 includes a fan-shaped pattern. The configuration of diffraction grating patterns 1700 in FIG. 17 includes a diagonal bar pattern. While only four examples are shown in FIGS. 14-17, it will be appreciated that the diffraction gratings suitable for use herein can include many configurations, including, but not to be limited to, longitudinal bars, concentric circles, concentric squares, concentric triangles, stars, discs, undulating waves, grids of squares, grids of triangles, grids of ovals, and the like.

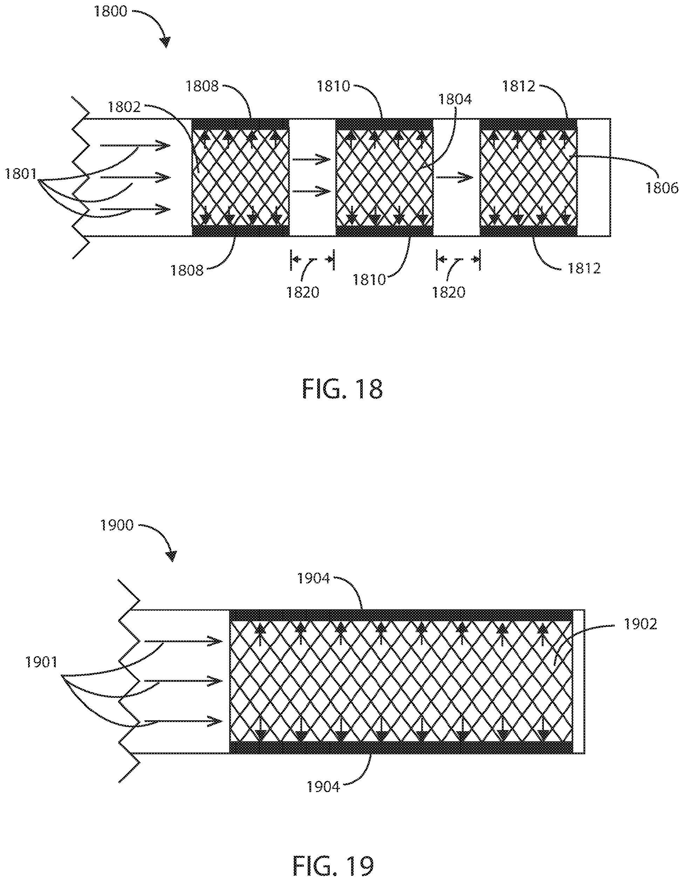

Light guides suitable for use in the embodiments herein can include one or more diffraction gratings and one or more fiber diffusers within the distal portion of the light guide to provide multiple selected regions within the light guide for directing toward the balloon surface. A fiber diffuser can be included as a part of the light guide that diverts light away from its axial path through the light guide and to a side surface portion. In some embodiments, the fiber diffusers can be included within the light guide at one or more regions of the distal portion. Referring now to FIG. 18 a schematic cross-sectional view of light guide 1800 is shown in accordance with various embodiments herein. Light guide 1800 includes a plurality of fiber diffusers including a first, second, and third fiber diffusers 1802, 1804, and 1806, respectively, positioned along the elongate shaft of the distal portion of the light guide 1800. Each fiber diffuser directs light 1801 from the light guide 1800 to exit the light guide 1800 at a side surface portion thereof. The side surface portions of the light guide 1800 are in optical communication with one or more diffraction gratings, such that the fiber diffusers and the diffraction gratings are in optical communication with one another.

By way of example, light guide 1800 includes a plurality of diffraction gratings including a first, second, and third diffraction gratings 1808, 1810, and 1812, respectively, positioned along the elongate shaft of the light guide 1800. The first, second, and third diffraction gratings 1808, 1810, and 1812, respectively, can be in optical communication with the first, second, and third fiber diffusers 1802, 1804, and 1806, respectively, at a plurality of side surface portions of light guide 1800. Light 1801 within each of the first, second, and third fiber diffusers 1802, 1804, and 1806 is directed to exit the light guide 1800 at a side surface portion and is dispersed by the first, second, and third diffraction gratings 1808, 1810, and 1812, respectively. Light energy can be dispersed by the diffraction gratings in a variety of ways depending on the configuration of the diffraction coating. The diffraction gratings 1808, 1810, and 1812 of light guide 1800 can be axially spaced apart with at least one intervening non-emitting portion 1820 of the light guide 1800 disposed between the plurality of diffraction gratings.

The fiber diffusers and diffraction gratings shown in FIG. 18 include those having a cylindrical shape. By way of example, the fiber diffusers 1802, 1804, and 1806 are configured to span the entire circumference of light guide 1800, and as such, the fiber diffusers 1802, 1804, and 1806 are cylindrical fiber diffusers. The diffraction gratings 1808, 1810, and 1812 are configured to span the entire circumference of light guide 1800, and as such, diffraction gratings 1808, 1810, and 1812 are cylindrical gratings.

In some embodiments, a single large fiber diffuser can be included within the light guide at the distal portion. Referring now to FIG. 19 a schematic cross-sectional view of light guide 1900 is shown in accordance with various embodiments herein. Light guide 1900 includes a single fiber diffuser 1902 positioned along the elongate shaft of the distal region of the light guide 1900. The fiber diffuser 1902 directs light 1901 to exit the light guide 1900 at a side surface portion thereof. The side surface portion of the light guide 1900 is in optical communication with diffraction grating 1904 and fiber diffuser 1902, such that the fiber diffuser 1902 and the diffraction grating 1904 are in optical communication with one another.

Light 1901 is dispersed by the diffraction grating 1904 about the light guide 1900. The fiber diffuser 1902 and the diffraction grating 1904 can be configured to span the entire circumference of light guide 1900. The fiber diffuser 1902 can be a cylindrical fiber diffuser. The diffraction grating 1904 can be a cylindrical grating. The side surface portion of the light guide disposed in between the fiber diffuser 1902 and the diffraction grating 1904 can be a cylindrical side portion. While light guides 1800 and 1900 are shown having both fiber diffusers and diffraction gratings, it will be appreciated that in some embodiments the light guides herein can include only fiber diffusers. In some embodiments, the light guides herein can include those with only diffraction gratings. In other embodiments, the light guides herein can include those having any combination of fiber diffusers and diffraction gratings, or both.

To create diffraction gratings along the light guide, at least a portion of the cladding is removed or modified to provide an opening in the cladding that exposes a region of the light guide core for placement of a diffraction grating. In some embodiments, the portion of the cladding that is removed can be in the shape of a square, a circle, an oval, a trapezoid, and the like. In some embodiments, the portion of the cladding removed from the light guide can extend circumferentially about the entire light guide. In other embodiments, the portion of the cladding removed from the light guide can extend circumferentially about a portion of the light guide. In some embodiments, the diffraction grating is integral to a cladding layer about the light guide. In yet other embodiments, the portion of the cladding removed from the light guide can be in the shape of a spiral circumferentially disposed about a portion of the light guide.

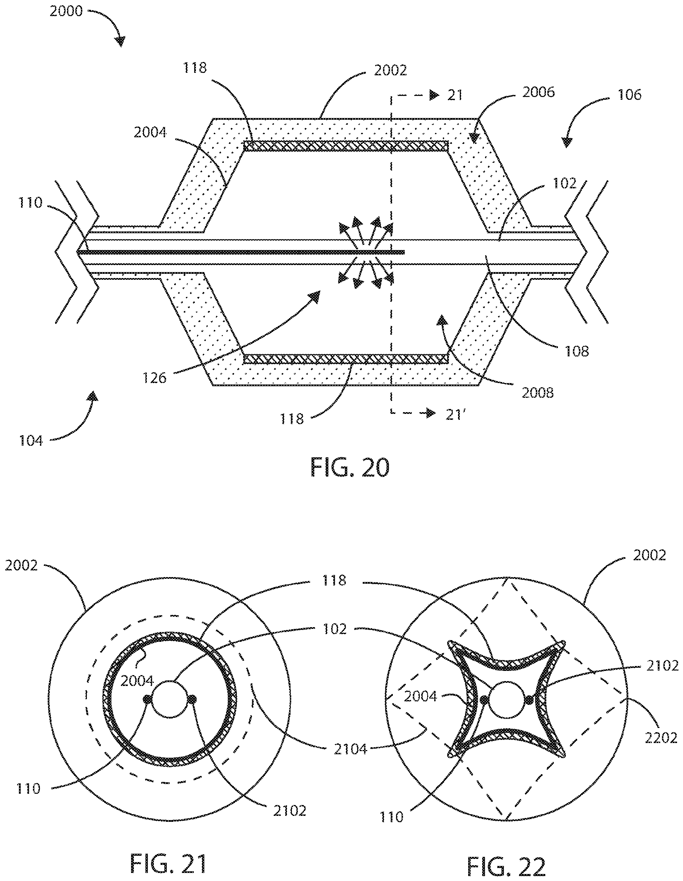

It will be appreciated that the photoacoustic catheters herein can include multiple balloons. Referring now to FIG. 20, a schematic cross-sectional view of a photoacoustic catheter 2000 is shown in accordance with various embodiments herein. Photoacoustic catheter 2000 can be adapted for placement within a blood vessel having a vessel wall. The photoacoustic catheter 2000 can be used to treat a vascular lesion found within or adjacent to the vessel wall. In some embodiments, the vascular lesion can include a calcified vascular lesion. In some embodiments, the vascular lesion can include a fibrous vascular lesion. The photoacoustic catheter 2000 can include an elongate shaft 102 extending from a proximal region 104 to a distal region 106, and can also include a lumen 108. In some embodiments, the photoacoustic catheter 2000 can have a distal region opening and can accommodate and be tracked over a guide wire to a treatment location. In some embodiments, the photoacoustic catheter 2000 does not include a lumen. In embodiments where the elongate shaft 102 does not include a lumen to be accessed by a caregiver, the elongate shaft 102 can be configured to allow the catheter to be steered through a patient's vasculature. For example, a wire extension at a distal region of the elongate shaft can improve steerability.

The elongate shaft 102 of photoacoustic catheter 2000 can be coupled to a first light guide 110 in optical communication with a light source 116. In some embodiments, the first light guide can be an optical fiber and the light source can be a laser. The light source 116 can be in optical communication with the first light guide 110 at a proximal region 104 of the elongate shaft 102. A schematic depiction of exemplary emitted light 126 as transmitted by the first light guide 110 is shown. It will be appreciated that, photoacoustic catheter 2000 can include more than one light guide. In some embodiments, photoacoustic catheter 2000 can include a second light guide, a third light guide, a fourth light guide, a fifth light guide, a sixth light guide, or more. In some embodiments, a plurality of light guides will be evenly spaced and radially offset from each other so that where there are *n* light guides, they are spaced apart by 360 degrees divided by *n.* In other embodiments, the light guides will be unevenly spaced and radially offset from each other.

It will be appreciated that the photoacoustic catheters herein can include any number of light guides. For example, in some embodiments, the photoacoustic catheters herein can include from one light guide to five light guides. In other embodiments, the photoacoustic catheters herein can include from five to fifteen light guides. In yet other embodiments, the photoacoustic catheters herein can include from ten light guides to thirty light guides. The photoacoustic catheters herein can include one, two, three, four, five, six, seven, eight, nine, or ten light guides. The photoacoustic catheters can include 11, 12, 13, 14, 15, 16, 17, 18, 19, 20, 21, 22, 23, 24, 25, 26, 27, 28, 29, or 30 light guides. It will be appreciated that photoacoustic catheters herein can include any number of light guides that can fall within a range, wherein any of the forgoing numbers can serve as the lower or upper bound of the range, provided that the lower bound of the range is a value less than the upper bound of the range. In some embodiments, the photoacoustic catheters herein can include more than 30 light guides.

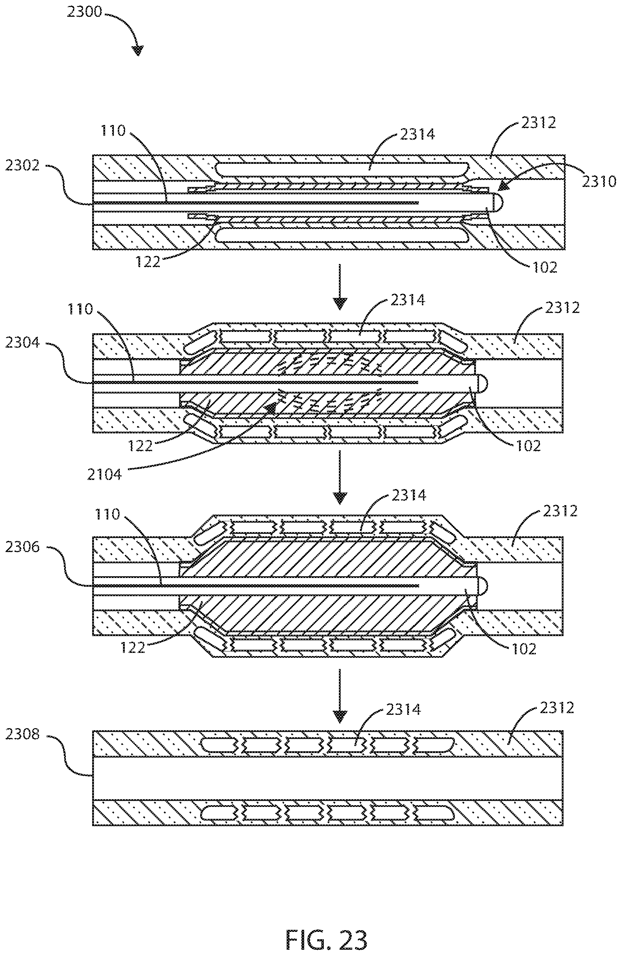

The photoacoustic catheter 2000 includes an outer balloon 2002 coupled to the elongate shaft 102. The outer balloon 2002 can expand from a collapsed configuration 2302 (illustrated in Figure 23) suitable for advancing the catheter through a patient's vasculature to a first expanded configuration 2304 (illustrated in Figure 23) suitable for anchoring the catheter in position relative to a treatment site. The photoacoustic catheter 2000 also includes an inner balloon 2004 coupled to the elongate shaft 102. The inner balloon 2004 can expand from a collapsed configuration suitable for advancing the catheter through a patient's vasculature to a first expanded configuration. In some embodiments, the outer balloon 2002 and/or inner balloon 2004 are configured to change from a first expanded configuration to a second, further expanded configuration. It will be appreciated that the balloons herein can assume multiple expanded configurations between a first expanded configuration and a second further expanded configuration. In some embodiments, the balloons herein will include a maximum expanded configuration. Expansion of the balloons herein to various expanded configurations will be discussed in more detail below.

The inner balloon 2004 of photoacoustic catheter 2000 can include a photoacoustic transducer 118 disposed on a surface of the inner balloon 2004 and can be in optical communication with the first light guide 110. In some embodiments, the photoacoustic transducer 118 is disposed on an outer surface of the inner balloon 2004, as shown in FIG. 20. In other embodiments, the photoacoustic transducer 118 is disposed on an inner surface of the inner balloon 2004, as discussed in reference to FIG. 3.

The photoacoustic transducer 118 disposed on the inner balloon 2004 can be adapted to impart acoustic pressure waves upon a calcified lesion to induce fractures in the calcified lesion. The photoacoustic transducer 118 can be formed from a light-absorbing material and a thermal expansion material. In some embodiments, the thermal expansion material of the photoacoustic transducer 118 is a polymer, and the polymer is in thermal contact with the light absorbing material. The thermal expansion material and the light absorbing material will both be discussed in more detail below.

The photoacoustic catheter 2000 can be configured to expand the outer balloon 2002 using an outer balloon inflation fluid 2006 and configured to expand the inner balloon 2004 using an inner balloon inflation fluid 2008. The balloon inflation fluids herein can include a fluid suitable for transmitting acoustic energy from the first light guide 110 to an inside surface of the outer balloon 2002 and the inner balloon 2004. Exemplary balloon inflation fluids can include, but are not to be limited to, water, saline, contrast agent, gases such as oxygen and carbon dioxide, and the like, and will be discussed in more detail below. In some embodiments, the outer balloon inflation fluid 2006 is a liquid and the inner balloon inflation fluid 2008 is a gas. In some embodiments, the outer balloon inflation fluid 2006 is a gas and the inner balloon inflation fluid 2008 is a liquid. In some embodiments the inner balloon inflation fluid 2008 is the same as the outer balloon inflation fluid 2006. In some embodiments, an acoustic impedance mismatch can exist between inner balloon inflation fluid 2008 and outer balloon inflation fluid 2006 to increase acoustic coupling efficiency.

Referring now to FIGS. 21 and 22, cross-sectional views of photoacoustic catheter 2000 along line 21-21' of FIG. 20 are shown in accordance with various embodiments herein. The photoacoustic catheter 2000 includes the outer balloon 2002, the inner balloon 2004, elongate shaft 102, a first light guide 110, a second light guide 2102, and a photoacoustic transducer 118 disposed about the outer surface of the inner balloon 2004. In some embodiments, the photoacoustic transducer 118 can be disposed about the inner surface of inner balloon 2004.

The cross-sectional view presented in FIG. 21 shows an inner balloon 2004 with a symmetrical shape and photoacoustic transducer 118 when in a first expanded configuration. The photoacoustic transducer 118 can be activated by a light source 116 in optical communication with the first light guide 110, the second light guide 2102, and the photoacoustic transducer 118 to generate at least one acoustic pressure wave 2104. The cross-sectional view presented in FIG. 22 shows an inner balloon 2004 with an asymmetrical shape and photoacoustic transducer 118 having a concave portion in a first expanded configuration. The photoacoustic transducer 118 can be activated by a light source 116 in optical communication with the first light guide 110, the second light guide 2102, and the photoacoustic transducer 118 to generate at least one acoustic pressure wave 2104 having at least one focal point 2202 on an inner surface of the outer balloon 2002. While the embodiments shown in FIGS. 21 and 22 include only a first light guide and a second light guide, it will be appreciated that multiple light guides can be used in photoacoustic catheter 2000, as discussed elsewhere herein.

It will be appreciated that the configuration of inner balloon 2004 of FIG. 22 can be further used to focus light to a specific location along a vascular lesion. While the configuration of FIG. 22 shows four concave portions for inner balloon 2004, it will be appreciated that the inner balloon can be configured to include from two to ten focusing elements disposed about the elongate shaft. In some embodiments, the inner balloon can be configured to include more than ten focusing elements about the elongate shaft. The focusing elements can be disposed uniformly or non-uniformly about the elongate shaft depending on the shape, size, and location of the vascular lesion to be treated. For example, for circumferential lesions, the inner balloon 2004 could include from two to ten focusing elements distributed evenly about the elongate shaft. For non-circumferential lesions, the inner balloon 2004 could include from two to ten focusing elements distributed non-uniformly about the elongate shaft. The acoustic focusing elements suitable for use herein can include a hydrophobic coating on the surface of the balloon, a honeycomb-like structure filled with gas or gas bubbles, a concave balloon-shaping structure made from nitinol, magnesium alloys, titanium alloys, biocompatible polymers, and the like. It will be further appreciated that the focusing elements of FIG. 22 can be present on a single balloon without an additional outer balloon present.

### Methods

The photoacoustic catheters described herein can be used in one or more methods for photoacoustically generating pressure waves within a blood vessel. Referring now to FIG. 23, a schematic flow diagram for a method 2300 is shown in accordance with the various embodiments herein. Method 2300 includes advancing a photoacoustic catheter 2310 to a treatment site 2314 within the blood vessel 2312, the photoacoustic catheter 2310 including an elongate shaft 102, a balloon 122 coupled to the elongate shaft 102, a first light guide 110, and a photoacoustic transducer 118 (not shown) disposed on a surface of the balloon 122 at 2302. The treatment site 2314 can include a vascular lesion within a patient's vasculature. In some embodiments, the vascular lesion can include a calcified lesion. In some embodiments, the vascular lesion can include a fibrous vascular lesion. The method 2300 includes expanding the balloon 122 from a collapsed configuration suitable for advancing the catheter through a patient's vasculature to a first expanded configuration 2304 suitable for anchoring the catheter in position relative to the treatment site 2314 at 2304. The method 2300 includes, after expanding the balloon 122, activating a light source 116 in optical communication with the first light guide 110 and the photoacoustic transducer 118, thereby imparting acoustic pressure waves 2104 upon the treatment site 2314 at 2304. The method 2300 can also include, after activating the light source, further expanding the balloon 122 from the first expanded configuration 2304 to a second further expanded configuration at 2306. The method can include completely removing the photoacoustic catheter 2310 from the patient's vasculature at 2308.

### Balloons

The balloons suitable for use in the photoacoustic catheters herein include those that exhibit good adhesion to the photoacoustic transducer materials described herein. For embodiments where the photoacoustic transducer is disposed on the exterior surface of the balloon, suitable balloon materials exhibit a high transparency for light. Without being bound by any particular theory, transparency can refer to the ability of a material to transmit light without appreciable scattering of the light by the material and is reported as total transmittance. Transmittance is reported as the ratio of transmitted light to the incident light, and can be reduced by reflection of light by the material, scattering of light by the material, and absorption of light by the material. In some embodiments, the materials suitable for use in the balloons herein can include those with a transmittance of about 50% to about 100%. In some embodiments, the transmittance can be greater than or equal to 50%, 60%, 70%, 80%, 90%, or 100%, or can be an amount falling within a range between any of the foregoing.

The balloons herein can be configured to be expanded from a collapsed configuration suitable for advancing the catheter through a patient's vasculature to a first expanded configuration suitable for anchoring the catheter in position relative to a treatment site. After treatment at a treatment site, the balloon can be further expanded from the first expanded configuration to a second further expanded configuration. In some embodiments, the second further expanded configuration includes a balloon diameter larger than the balloon diameter of the first expanded configuration. It will be appreciated that the balloons herein can assume multiple expanded configurations between the first expanded configuration and a second further expanded configuration. In some embodiments, the balloons herein will include a maximum expanded configuration. In some embodiments, the second further expanded configuration can be the maximum expanded configuration of the balloon.

In some embodiments, the balloons herein are made from silicone. In other embodiments, the balloons herein are made from polydimethylsiloxane (PDMS), polyurethane, polymers such as PEBAX^{™} material available from Arkema (which has a location at King of Prussia, Pennsylvania, USA), nylon, and the like. In some embodiments, the balloons can include those having diameters ranging from 1 millimeter (mm) to 25 mm in diameter. In some embodiments, the balloons can include those having diameters ranging from 1.5 mm to 12 mm in diameter. In some embodiments, the balloons can include those having diameters ranging from 1 mm to 5 mm in diameter. In some embodiments, the diameter can be greater than or equal to 0.5 mm, 1.0 mm, 1.5 mm, 2.0 mm, 2.5 mm, 3.0 mm, 3.5 mm, 4.0 mm, 4.5 mm, 5.0 mm, 5.5 mm, 6.0 mm, 6.5 mm, 7.0 mm, 7.5 mm, 8.0 mm, 8.5 mm, 9.0 mm, 9.5 mm, 10.0 mm, 10.5 mm, 11.0 mm, 11.5 mm, 12.0 mm, 12.5 mm, 13.0 mm, 13.5 mm, 14.0 mm, 14.5 mm, 15.0 mm, 15.5 mm, 16.0 mm, 16.5 mm, 17.0 mm, 17.5 mm, 18.0 mm, 18.5 mm, 19.0 mm, 19.5 mm, or 20.0 mm, or can be an amount falling within a range between any of the foregoing.

In some embodiments, the balloons herein can include those having a length ranging from 5 mm to 300 mm in length. In some embodiments, the balloons herein can include those having a length ranging from 8 mm to 200 mm in length. In some embodiments, the length of the balloon can be greater than or equal to 5 mm, 10 mm, 20 mm, 30 mm, 40 mm, 50 mm, 60 mm, 70 mm, 80 mm, 90 mm, 100 mm, 110 mm, 120 mm, 130 mm, 140 mm, 150 mm, 160 mm, 170 mm, 180 mm, 190 mm, 200 mm, 210 mm, 220 mm, 230 mm, 240 mm, 250 mm, 260 mm, 270 mm, 280 mm, 290 mm, or 300 mm, or can be an amount falling within a range between any of the foregoing.

The balloons herein can be inflated to inflation pressures from 1 atmosphere (atm) to 70 atm. In some embodiments, the balloons herein can be inflated to inflation pressures of from 6 atm to 20 atm. In some embodiments, the balloons herein can be inflated to inflation pressures of from 20 atm to 70 atm. In some embodiments, the balloons herein can be inflated to inflation pressures that can be greater than or equal to 1 atm, 10 atm, 20 atm, 30 atm, 40 atm, 50 atm, 60 atm, or 70 atm, or can be an amount falling within a range between any of the foregoing.

The balloons herein can include those having various shapes, including, but not to be limited to, a conical shape, a square shape, a rectangular shape, a spherical shape, a conical/square shape, a conical/spherical shape, an extended spherical shape, an oval shape, a tapered, shape, a bone shape, a stepped diameter shape, an offset shape, or a conical offset shape.

In some embodiments, the balloons herein can provide a therapeutic agent to a treatment site. In some embodiments, the therapeutic agent can be delivered via a drug eluting coating, a drug eluting stent structure, or by the delivery of a drug composition through one or more lumens of the catheters described herein. The drug elution coating or drug eluting stent structure can include one or more therapeutic agents including anti-inflammatory agents, anti-neoplastic agents, anti-angiogenic agents, and the like. Exemplary agents can include, but is not to be limited to paclitaxel, docetaxel, everolimus, and sirolimus, and analogs thereof.

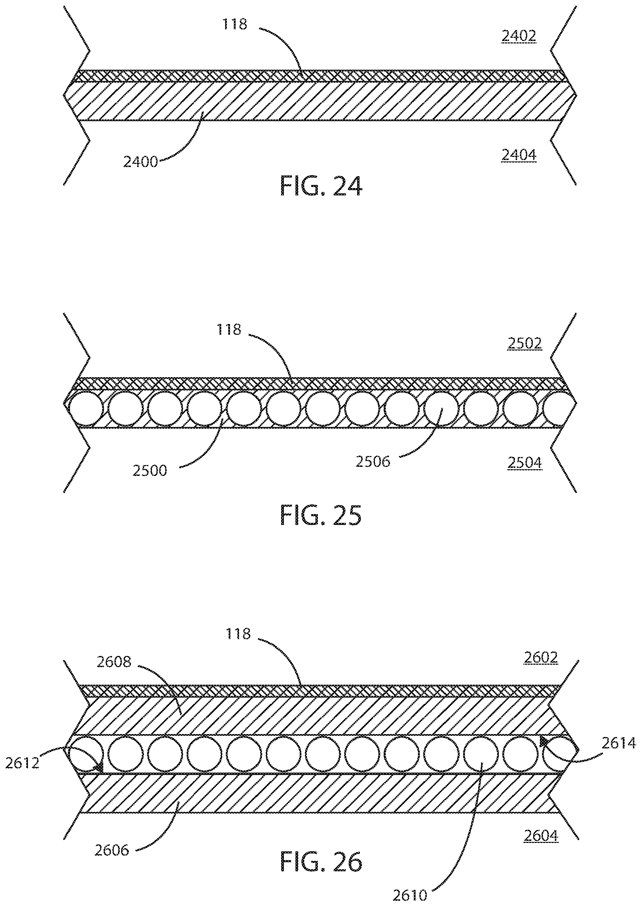

The balloons suitable for use in the photoacoustic catheters herein can include one or more acoustic focusing elements, such as an acoustic mirror. Referring now to FIGS. 24-25, schematic cross-sectional views of a balloon are shown in accordance with various embodiments herein. In the configuration shown in FIG. 24, a balloon 2400 includes an outer surface 2402 that can be exposed to the luminal wall of a vessel within a patient and an inner surface 2404 that is disposed on the interior of the balloon 2400. Balloon 2400 includes a photoacoustic transducer 118 disposed on an outer surface of a balloon 2400. The balloon 2400 does not include an acoustic focusing element. In the configuration shown in FIG. 25, a balloon 2500 includes an outer surface 2502 that can be exposed to the luminal wall of a vessel within a patient and an inner surface 2504 that is disposed on the interior of the balloon 2500. Balloon 2500 includes a photoacoustic transducer 118 disposed on an outer surface of the balloon 2500, and further includes a plurality of gas bubbles 2506 integrated within the balloon material of balloon 2500. The plurality of gas bubbles 2506 can act as an acoustic focusing element, such as an acoustic mirror, used to focus the acoustic pressure wave at any given location(s) on the vessel wall of a patient to treat a vascular lesion.

Embodiments herein having multiple balloons can also be configured to include one or more focusing elements. By way of example, two balloon walls can be configured to trap gas therebetween such that the trapped gas can act as an acoustic mirror. In the configuration shown in FIG. 26, the photoacoustic catheter can include an inner balloon 2606 and an outer balloon 2608. The outer balloon 2608 can include an outer surface 2602 that can be exposed to the luminal wall of a vessel within a patient and an inner surface 2614 facing the inner balloon 2606. The inner balloon 2606 can include an inner surface 2604 that is disposed on an interior portion of the photoacoustic catheter and exposed to a balloon fluid, and an outer surface 2612 facing the outer balloon 2608. The outer balloon 2608 can include a photoacoustic transducer 118 disposed on the outer surface of the outer balloon 2608. The inner balloon 2606 and the outer balloon 2608 can be configured to trap a plurality of gas bubbles 2610 in between the inner surface 2614 of outer balloon 2608 and the outer surface 2612 of inner balloon 2606 when in an expanded configuration. In one example, the outer balloon 2608 has smooth surfaces on both the outer surface 2602 and inner surface 2614, while the inner balloon 2606 has a smooth inner surface 2604 and features or structures on the outer surface 2612 so that when inflated against the outer balloon, gas is trapped in the features or structures to create an acoustic mirror. The plurality of gas bubbles 2610 trapped between the inner balloon 2606 and outer balloon 2608 can act as an acoustic focusing element used to focus the acoustic pressure wave at any given location(s) on the vessel wall of a patient to treat a vascular lesion. The acoustic focusing elements suitable for use on a surface of the inner balloon 2606 or outer balloon 2608 can further include a hydrophobic coating a honeycomb-like structure filled with gas or gas bubbles, a concave balloon-shaping structure made from nitinol, magnesium alloys, titanium alloys, biocompatible polymers, and the like.

### Balloon Fluids

Exemplary balloon fluids suitable for use herein can include, but are not to be limited to one or more of water, saline, contrast agent, fluorocarbons, perfluorocarbons, gases, such as carbon dioxide, and the like. In some embodiments, the balloon inflation fluids include a mixture of saline to contrast agent in a volume ratio of 50:50. In some embodiments, the balloon fluids include a mixture of saline to contrast agent in a volume ratio of 25:75. In some embodiments, the balloon fluids include a mixture of saline to contrast agent in a volume ratio of 75:25. The balloon fluids suitable for use herein can be tailored on the basis of composition, viscosity, and the like in order to manipulate the rate of travel of the acoustic pressure waves therein. The balloon fluids suitable for use herein are biocompatible.

In some embodiments, the contrast agents used herein can include, but are not to be limited to, iodine-based contrast agents, such as ionic or non-ionic iodine-based contrast agents. Some non-limiting examples of ionic iodine-based contrast agents include diatrizoate, metrizoate, iothalamate, and ioxaglate. Some non-limiting examples of non-ionic iodine-based contrast agents include iopamidol, iohexol, ioxilan, iopromide, iodixanol, and ioversol. In other embodiments, non-iodine based contrast agents can be used. Suitable non-iodine containing contrast agents can include gadolinium (III)-based contrast agents. Suitable fluorocarbon and perfluorocarbon agents can include, but are not to be limited to, agents such as the perfluorocarbon dodecafluoropentane (DDFP, C5F12), perfluoro-octane (PFO), perfluoroperhydrophenanthrene, perfluorodecalin (PFD), perfluorotributylamide (PFTB) and perfluorooctylbromide (PFOB), and the like.

### Photoacoustic Transducers

The photoacoustic transducers herein can include those that can be disposed on the internal surface of a balloon or the external surface of a balloon. The photoacoustic transducers disposed on a surface of the balloon can include those that are configured to span the entire circumference of balloon. In some embodiments, the photoacoustic transducers can also include partial transducers that are configured to span from at least 1 to 359 degrees about the balloon in a circumferential direction. In some embodiments, the photoacoustic transducers herein can be disposed on a surface of the balloon in one or more photoacoustic transducer patterns as described herein. By way of example, the photoacoustic transducer patterns can include, but are not to be limited to, circumferential bars, longitudinal bars, diagonal bars, or islands of various shapes and sizes. In various embodiments herein, the photoacoustic catheters can include a plurality of photoacoustic transducers, including a first photoacoustic transducer, a second photoacoustic transducer, a third photoacoustic transducer, a fourth photoacoustic transducer, a fifth photoacoustic transducer, a sixth photoacoustic transducer, a seventh photoacoustic transducer, an eighth photoacoustic transducer, a ninth photoacoustic transducer, a tenth photoacoustic transducer, etc.

Without wishing to be bound by any particular theory, the photoacoustic transducer patterns having multiple photoacoustic transducers herein can be tailored to generate acoustic pressure waves such that adjacent photoacoustic transducers generate acoustic pressure waves that can constructively interfere with one another to generate higher peak pressure at a given location within the vascular lesion.

The photoacoustic transducers herein can generate acoustic pressure waves upon the site of a calcified lesion. The acoustic pressure waves generated by the photoacoustic transducers can be tailored for the specific application, including directionality, shape, convergence, or divergence by tailoring the size, shape and excitation of the photoacoustic transducer(s) and the balloon surface. In some embodiments, the photoacoustic transducer(s) and balloon surface can be tailored to generate a cylindrical acoustic wave symmetrically about a balloon. In other embodiments, the photoacoustic transducers can be tailored to generate multiple acoustic pressure waves from more than one location on a surface of a balloon. In other embodiments, the photoacoustic transducers can be tailored to generate multiple acoustic pressure waves that are directionally offset from one another by from zero degrees to 180 degrees about the balloon.

The size of the photoacoustic transducers can vary and can depend on the treatment location to be accessed. In some embodiments, the photoacoustic transducers can be from 0.1 mm to 6 mm, 0.1 mm to 2 mm, or 10 mm to 100 mm in diameter or width. In some embodiments, the photoacoustic transducers can be from 5 mm to 30 mm in diameter or width. In some embodiments, the photoacoustic transducers can be from 10 mm to 20 mm in diameter or width. A size for a single photoacoustic transducer of about 1 mm² or less is possible and has efficiency advantages over larger photoacoustic transducers. In other embodiments, the photoacoustic transducers can collectively span from 0.1 mm to 6 mm, 0.1 mm to 2 mm, 10 mm to 100 mm, from 5 mm to 30 mm along the length of the balloon. In yet other embodiments, the photoacoustic transducers can exceed 30 mm in length, exceed 100 mm in length, such as in the context of coronary treatment, or exceed 200 mm to 300 mm in length, such as in the context of peripheral vascular treatment.

The photoacoustic transducers herein can include a light-absorbing material and a thermal expansion material. Exemplary light absorbing material suitable for use herein can include strong light-absorbing materials having large absorption coefficients of units inverse centimeters. Some exemplary light-absorbing materials can include, but not be limited to, nanoparticles, carbon nanotubes, candle soot, candle soot nanoparticles, carbon black, a nanotube array, multiwall carbon nanotubes, and light absorbing dyes. The light-absorbing materials herein can be highly absorbing of laser light such that absorption is rapid on the nanosecond timescale. The rapid absorption of light energy by the light-absorbing material can enable the efficient transfer of thermal energy to the thermal expansion material, thus driving the generation of acoustic waves.

Thermal expansion materials suitable for use herein can include materials having a strong coefficient of thermal expansion. For example, the thermal expansion material can have a coefficient of thermal expansion from 23 degrees Celsius to 100 degrees Celsius of about 0.000012 1/K or higher, about 0.0001 1/K or higher, 0.0002 1/K or higher, or about 0.0003 1/K or higher.

Suitable thermal expansion materials can include polymers having a strong coefficient of thermal expansion. Examples of suitable materials include, but are not to be limited to, polydimethylsiloxane (PDMS), polytetrafluoroethylene (PTFE), polyimide, polyisobutylene (PIB), PIB polyurethane, polyurethanes, styrene isoprene butadiene, ethylene propylene polyacrylic, ethylene acrylic, fluorosilicone, polybutadiene, polyisoprene, and thermoplastic elastomers. For a silicone PDMS material, the coefficient of thermal expansion from 23 degrees Celsius to 100 degrees Celsius can be about 0.00034 1/K. For a plain PTFE material, the coefficient of thermal expansion from 23 degrees Celsius to 100 degrees Celsius can be about 0.000143 1/K. For a polyimide material, the coefficient of thermal expansion from 23 degrees Celsius to 100 degrees Celsius can be about 0.00014 1/K.

Thermal expansion materials suitable for use herein can also include thin metallic films. Thin metallic films can be used alone, or in combination with additional thermal expansion materials, such as thermal expansion materials having a high coefficient of thermal expansion (CTE). Some exemplary metals for use as thermal expansion materials in thin metallic films include, but are not to be limited to silver, copper, and gold, aluminum, beryllium, tungsten, and magnesium.

The photoacoustic transducers herein can be formed by layering a light-absorbing material and a thermal expansion material on a surface of the balloons described herein. In some embodiments, the light-absorbing material and a thermal expansion material can form a composite film on or around the balloons described herein. In some embodiments, the composite film can include one that has layers of the light-absorbing material. In one embodiment, a layer of the light-absorbing material can be disposed on a balloon in optical contact with the core of the light guide and a thermal expansion material can be disposed on the surface of the light-absorbing material layer at the outermost surface. In some embodiments, the thermal expansion material is in thermal contact with the light absorbing material. In some embodiments, the thermal expansion material is in direct contact with the light absorbing material. In other embodiments, the thermal expansion material is in a matrix with the light absorbing material. In yet other embodiments, the thermal expansion material and the light absorbing material are the same.

One suitable configuration for the light-absorbing material and a thermal expansion material can include a layer of candle-soot nanoparticles as the light-absorbing material in contact with a layer of polydimethylsiloxane as the thermal expansion material. Another suitable configuration for the light-absorbing material and a thermal expansion material can include a layer of multiwall carbon nanotubes as the light-absorbing material in contact with a layer of polydimethylsiloxane as the thermal expansion material.

The light-absorbing material and a thermal expansion material can be applied to the balloons using various techniques. In some embodiments, the light-absorbing material and a thermal expansion material can be individually applied to the balloons using a spray coating technique. In other embodiments, the light-absorbing material and a thermal expansion material can be individually applied to the balloons using a dip coating technique. In yet other embodiments, the light-absorbing material and a thermal expansion material can be individually applied to the balloons using an e-spun coating technique.

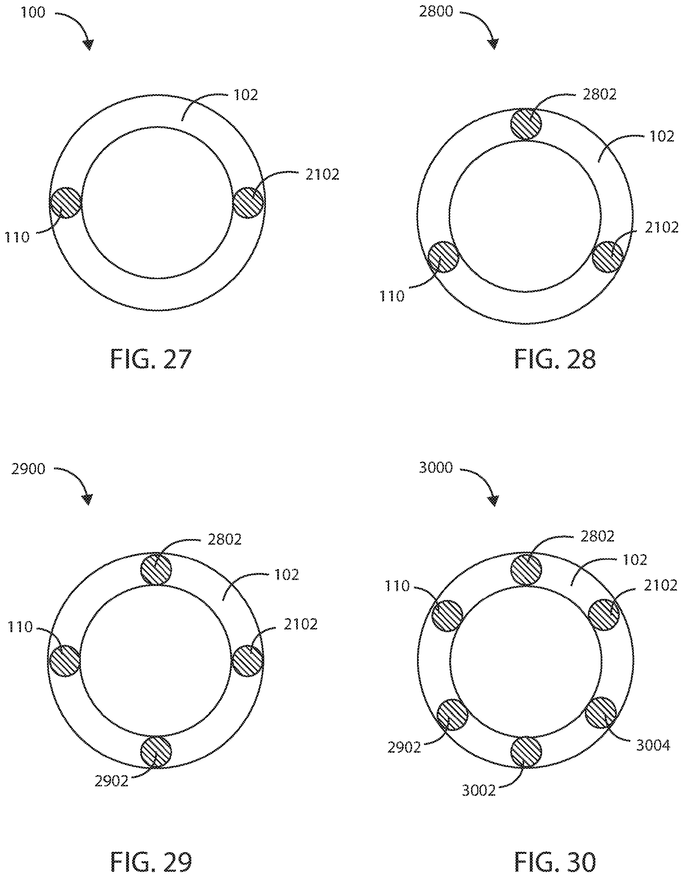

### Light Guides (FIGS. 27-30 and 43-45)

The light guides herein can include an optical fiber or flexible light pipe. The light guides herein can be thin and flexible and can allow light signals to be sent with very little loss of strength. The light guides herein can include a core surrounded by a cladding about its circumference. In some embodiments, the core can be a cylindrical core or a partially cylindrical core. The core and cladding of the light guides can be formed from one or more materials, including but not limited to one or more types of glass, silica, or one or more polymers. The light guides may also include a protective coating, such as a polymer. It will be appreciated that the index of refraction of the core will be greater than the index of refraction of the cladding.

Each light guide can guide light along its length to a distal portion having a photoacoustic transducer connected thereto. The light guide can create a light path as a portion of an optical network including a light source. The light path within the optical network allows light to travel from one part of the network to another without being modified. Both the optical fiber or the flexible light pipe can provide a light path within the optical networks herein.

The light guides herein can assume many configurations about the elongate shaft of the photoacoustic catheters described herein. In some embodiments, the light guides can run parallel to the longitudinal axis of the elongate shaft of the photoacoustic catheter. In some embodiments, the light guides can be disposed spirally or helically about the longitudinal axis of the elongate shaft of the photoacoustic catheter. In some embodiments, the light guides can be physically coupled to the elongate shaft. In other embodiments, the light guides can be disposed along the length of the outer diameter of the elongate shaft. In yet other embodiments the light guides herein can be disposed within one or more light guide lumens within the elongate shaft. Various configurations for the elongate shafts and light guide lumens will be discussed below.

Examples of photoacoustic catheters having multiple light guides disposed about the elongate shaft at different positions around the circumference are shown in FIGS. 27-30. Referring now to FIG. 27, a schematic cross-sectional view of a photoacoustic catheter 100 of FIG. 1 along line 27-27' in FIG. 1 is shown in accordance with various embodiments herein. Photoacoustic catheter 100 includes an elongate shaft 102, a first light guide 110 and a second light guide 2102 separated by about 180 degrees around the circumference. Referring now to FIGS. 28-30, schematic cross-sectional views of additional configurations for photoacoustic catheters having multiple light guides are shown in accordance with various embodiments herein. The configuration of photoacoustic catheter 2800 in FIG. 28 includes an elongate shaft 102, a first light guide 110, a second light guide 2102, and a third light guide 2802 separated by about 120 degrees around the circumference. The configuration of photoacoustic catheter 2900 in FIG. 29 includes an elongate shaft 102, a first light guide 110, a second light guide 2102, a third light guide 2802, and a fourth light guide 2902 separated by about 90 degrees around the circumference. The configuration of photoacoustic catheter 3000 shown in FIG. 30 includes an elongate shaft 102, a first light guide 110, a second light guide 2102, a third light guide 2802, a fourth light guide 2902, a fifth light guide 3002, and a sixth light guide 3004 separated by about 60 degrees around the circumference.

When multiple light guides are present, the light guides can be radially offset from one another by about at least about or about 45 degrees. In some embodiments, the light guides can be radially offset from one another by at least about or about 60 degrees. In some embodiments, the light guides can be radially offset from one another by about at least about or 90 degrees. In some embodiments, the light guides can be radially offset from one another by about 180 degrees. In some embodiments, a plurality of light guides will be evenly spaced and radially offset from each other so that where there are *n* light guides, they are spaced apart by 360 degrees divided by *n.* In some embodiments, each of the light guide locations shown in FIGS. 27-30 or otherwise described herein include two parallel light guides that are touching.

The light guides herein can include one or more diverting features configured to direct light within the light guide toward a side surface portion of the distal portion of the light guide. The diverting feature can include a reflecting element, a refracting element, a fiber diffuser, or any combination thereof, and a first light window positioned on the side surface portion. When light guides include a diverting feature configured to direct light within the light guide toward a side surface portion of the distal portion of the light guide, the light guides can also include at least a first light window positioned on a side surface portion of the light guide. In some embodiments the light windows span the entire circumference of the light guides, while in other embodiments the light windows only span a portion of the circumference of the light guides. Other properties of the light guides, including size, spacing, and distribution are described elsewhere herein.

In various embodiments, the light guides herein include one or more fiber diffusers. In some embodiments, a light guide can include a first fiber diffuser in a distal portion of the light guide, where the first fiber diffuser directs light from the light guide to exit the light guide at a side surface portion of the light guide. In cases where a light guide includes a first fiber diffuser to direct light from the light guide to exit the light guide at a side surface portion of the light guide, the side surface portion of the light guide is in optical communication with a first light window. In some embodiments the light windows span the entire circumference of the light guides, while in other embodiments the light windows only span a portion of the circumference of the light guides.

In yet other embodiments, the light guides herein can include a plurality of light windows and a plurality of fiber diffusers in the distal portion of the light guide. The plurality of light windows can include a first light window and the plurality of fiber diffusers can include the first fiber diffuser. Each fiber diffuser can direct light from the light guide to exit the light guide at a side surface portion of the light guide, where each side surface portion is in optical communication with one of the plurality of light windows. The plurality of light windows can be axially spaced apart with at least one intervening non-emitting portion of the light guide disposed between each of the plurality of light windows. The side surface portion can be a cylindrical side surface portion and a first light window can be configured as a cylindrical window.

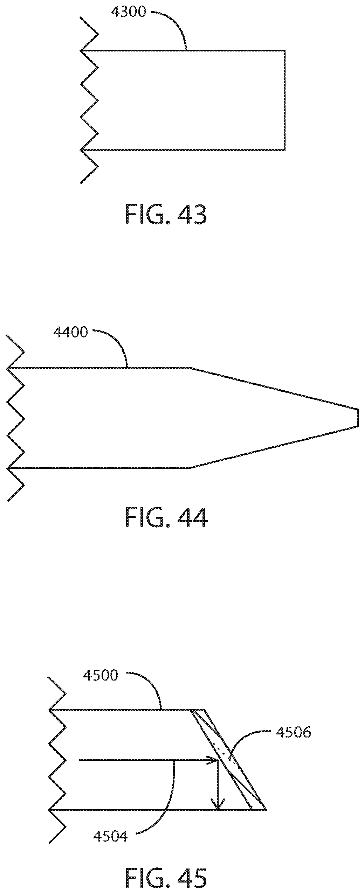

The light guides herein can include various configurations at a distal portion of the light guide. Referring now to FIGS. 43-45, schematic cross-sectional views of the distal portions of various shaped light guides are shown in accordance with various embodiments herein. In FIG. 43, a schematic cross-sectional view of a light guide 4300 is shown. Light guide 4300 includes a cylindrical end shape. In some embodiments, the end of the light guide can have a tapered shape. By way of example, in FIG. 44 a schematic cross-sectional view of a light guide 4400 having a tapered end shape is shown. In some embodiments, the end of the light guide can have an angled shape. By way of example, in FIG. 45 a schematic cross-sectional view of a light guide 4500 is shown. Light guide 4500 includes an angled end shape. The light guide 4500 also includes a diverting feature 4506 at the distal portion to direct the light 4504 within the light guide toward the side surface portion of the light guide. Light guide 4500 is configured such that light 4504 travels from a light source (not shown) in the direction from the proximal region of the light guide to the distal region of the light guide 4500, as indicated by the arrow. Upon contact with the diverting feature 4506, the light 4504 is diverted, or reflected, within the light guide 4500.

In some embodiments, a diverting feature can be included with the light guide to direct light toward a side surface portion of the distal portion of the light guide. A diverting feature can include any feature of the system herein that diverts light from the light guide away from its axial path toward a side surface portion of the light guide. Examples include a reflector, a refracting structure, and a fiber diffuser. Fiber diffusers will be discussed in more detail below.

In other embodiments, the light guides can form a spiral configuration about the longitudinal axis of the elongate shaft of the photoacoustic catheter. In some embodiments, the spiral configuration can run clockwise about the longitudinal axis of the elongate shaft of the photoacoustic catheter, while in other embodiments the spiral configuration can run counter-clockwise about the longitudinal axis of the elongate shaft of the photoacoustic catheter. In some embodiments, the light guides can form a single helix, a double helix, a triple helix, or a quadruple helix about the longitudinal axis of the elongate shaft of the photoacoustic catheter.

The light guides herein can come in various sizes and configurations. The light guides will have a longitudinal axis along the elongate shaft of the light guide and short axis about its circumference. In some embodiments, the light guides can have an outer diameter of about 100 µm, including the cladding and the core. In other embodiments, the light guides can include those that have an outer diameter of from 50 µm to 1000 µm including the cladding and the core. The length of the light guides can include those having a length of from 40 cm to 175 cm. In some embodiments, the length of the light guides can include those having a length of from 50-150 cm. In some embodiments, the length of the light guide can include those having a length of 40 cm, 50 cm, 60 cm, 70 cm, 80 cm, 90 cm, 100 cm, 125 cm, 150 cm, or 175 cm. It will be appreciated that the light guides herein can have a usable length that can fall within a range, wherein any of the forgoing lengths can serve as the lower or upper bound of the range, provided that the lower bound of the range is a value less than the upper bound of the range.

It will be appreciated that one or more light guides herein can be adhered to the outer surface of the elongate shaft of a catheter, to create a photoacoustic catheter. However, in other embodiments, one or more light guides can be disposed within a lumen of a photoacoustic catheter. In addition, the photoacoustic catheter may define a lumen for a guidewire having an inner diameter of about 0.014 inch (0.356 mm). In some embodiments, the photoacoustic catheter can include those having an inner diameter of about 0.018 inch (0.457 mm). In yet other embodiments, the photoacoustic catheter can include those having an inner diameter of about 0.035 inch (0.889 mm). In some embodiments the light guides herein can be integrated with a balloon catheter. In some embodiments the light guides herein can be integrated into a guide wire. In embodiments where the light guide is integrated into a guide wire, the resulting photoacoustic catheter can be used independently or can be used with various other balloon catheters.

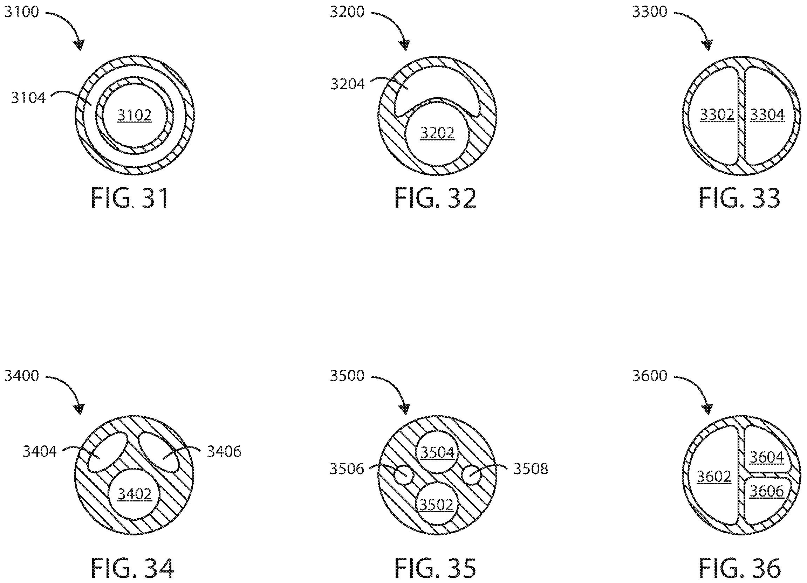

### Lumens of the Elongate Shaft (FIGS. 31-42)

The elongate shafts herein can include one or more lumens that span the length of the elongate shaft. Referring now to FIGS. 31-42, schematic cross-sectional views of various embodiments of an elongate shaft having multiple lumens are in accordance with various embodiments herein. In some embodiments, the elongate shaft can define a guidewire lumen. In some embodiments, the elongate shaft defines an inflation lumen surrounding the guidewire lumen, where the inflation lumen is in fluid communication with a balloon at a distal portion of the elongate shaft. In other embodiments, the elongate shaft defines an inflation lumen disposed alongside the guidewire lumen, where the inflation lumen is in fluid communication with a balloon at a distal portion of the elongate shaft. In yet other embodiments, the elongate shaft defines at least one control lumen and at least one light guide lumen.

In the configuration in FIG. 31, elongate shaft 3100 includes concentrically disposed guidewire lumen 3102 and an inflation lumen 3104. In the configuration in FIG. 32, elongate shaft 3200 includes guidewire lumen 3202 and an inflation lumen 3204 disposed adjacent to and partially surrounding guidewire lumen 3202. In the configuration in FIG. 33, elongate shaft 3300 includes guidewire lumen 3302 and an inflation lumen 3304 disposed adjacent to guidewire lumen 3302. In the configuration in FIG. 34, elongate shaft 3400 includes guidewire lumen 3402, inflation lumen 3404, and a control lumen 3406. It will be appreciated that any of the control lumens described herein can be used for many purposes, including, but not to be limited to, blood flow, cooling or heating fluid flow, delivery of a diagnostic or therapeutic agent, a light guide lumen, an inflation lumen, and the like. In the configuration in FIG. 35, elongate shaft 3500 includes guidewire lumen 3502, inflation lumen 3504, and two control lumens 3506 and 3508. In the configuration in FIG. 36, elongate shaft 3600 includes guidewire lumen 3602, inflation lumen 3604, and control lumen 3606.

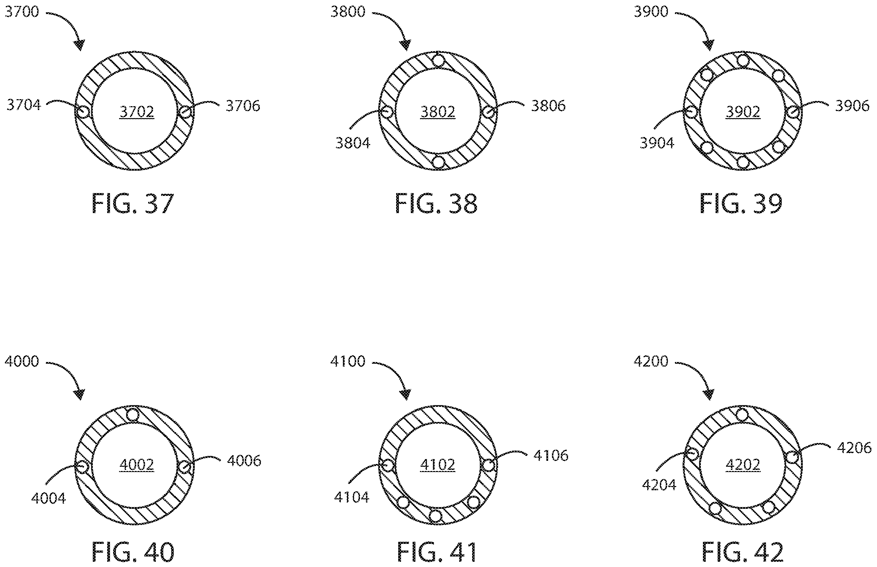

The light guides can be disposed within one or more light guide lumens disposed within the elongate shafts symmetrically about the circumference. In the configuration in FIG. 37, elongate shaft 3700 includes guidewire lumen 3702, light guide lumen 3704, and control lumen 3706. One or more of lumens 3702, 3704 and 3706 can serve as an inflation lumen. In the configuration in FIG. 38, elongate shaft 3800 includes guidewire lumen 3802, light guide lumen 3804, and control lumen 3806. Elongate shaft 3800 includes two additional lumens that can both be configured as light guide lumens, control lumens, or both a light guide lumen and control lumen. One or more of lumens 3802, 3804 and 3806 can serve as an inflation lumen. In the configuration in FIG. 39, elongate shaft 3900 includes guidewire lumen 3902, light guide lumen 3904, and control lumen 3906. Elongate shaft 3900 includes six additional lumens that can be configured as inflation lumens, light guide lumens, control lumens, or any combination of inflation lumens, light guide lumens and control lumens.

The light guides can be disposed within one or more light guide lumens disposed within the elongate shafts asymmetrically about the circumference. In the configuration in FIG. 40, elongate shaft 4000 includes guidewire lumen 4002, light guide lumen 4004, and control lumen 4006. Elongate shaft 4000 includes one additional lumen that can be configured as an inflation lumen, a light guide lumen or a control lumens. In the configuration in FIG. 41, elongate shaft 4100 includes guidewire lumen 4102, light guide lumen 4104, and control lumen 4106. Elongate shaft 4100 includes three additional lumens that can be configured as inflation lumens, light guide lumens, control lumens, or any combination of light guide lumens and control lumens. In the configuration in FIG. 42, elongate shaft 4200 includes guidewire lumen 4202, light guide lumen 4204, and control lumen 4206. Elongate shaft 4200 includes three additional lumens that can be configured as inflation lumens, light guide lumens, control lumens, or any combination of inflation lumens, light guide lumens and control lumens.

It will be appreciated that the lumens described in FIGS. 31-42 can assume many shapes, including, but not to be limited to, circular shape, square shape, crescent shape, triangular shape, and the like. The lumens of the elongate shafts can by symmetrically disturbed in the elongate shaft, asymmetrically distributed, or concentrically distributed. It will be further appreciated that the light guide lumens herein can be coated along the longitudinal length of the elongate shaft with a reflective material capable of propagating light along the elongate shaft from a distal light source to the proximal portion of the photoacoustic catheter.

### Fiber Diffusers

A fiber diffuser directs light from within a light guide to exit at a side surface portion of the light guide. The fiber diffusers described herein can be created several ways. In some embodiments, the fiber diffusers can be created by micro-machining the surface of the distal portion of a light guide with a CO₂ laser. In some embodiments, a fused silica coating can be applied to the distal portion of the light guide. In other embodiments, the fiber diffuser can be formed from a glass, a polymer, or a metal coating on the distal portion of the light guide. In other embodiments, the fiber diffuser can be formed by a fiber Bragg grating on the distal portion of the light guide. In some embodiments, the fiber diffuser can include a machined portion of the light guide, a laser-machined portion of the light guide, fiber Bragg gratings, a fused splicing, a fused splicing forming at least one internal mirror, and a splicing of two or more diffuse regions. Suitable materials for a fiber diffuser can include, but not be limited to, the materials of the core or cladding, ground glass, silver coated glass, gold coated glass, TiO2, and other materials that will scatter and not significantly absorbed the light wavelength of interest. One method that can be used to create a uniform diffuser in a light guide, optical component, or materials is to utilize scattering centers on the order of 50 nanometers to 5 micrometers in size. The scattering centers can have a distribution around 200 nanometers in size.

### Light Sources

The light sources suitable for use herein can include various types of light sources including lasers and lamps. Suitable lasers can include short pulse lasers on the nanosecond (ns) timescale. The lasers can also include short pulse lasers on the picosecond (ps), femtosecond (fs), and microsecond (us) timescales.

Exemplary nanosecond lasers can include those within the UV to IR spectrum, spanning wavelengths of about 10 nanometers to 10 millimeters. Nanosecond lasers can include those having repetition rates of up to 200 kHz. In some embodiments, the laser can include a Q-switched thulium:yttrium-aluminum-garnet (Tm:YAG) laser. In some embodiments, the laser can include a neodymium:YAG (Nd:YAG), holmium:YAG (Ho:YAG), erbium:YAG (Er:YAG), excimer laser, helium-neon laser, carbon dioxide laser, as well as doped, pulsed, fiber lasers.

### Acoustic Pressure Waves

The photoacoustic catheters herein can generate acoustic pressure waves having pressures in the range of 2 megapascals (MPa) to 25 MPa. The maximum pressure generated by a particular photoacoustic catheter will depend on the light source, the absorbing material, the propagation medium, a distance of the measurement device to the source of the pressure wave, and any other relevant factors. In some embodiments, the photoacoustic catheters herein can generate acoustic pressure waves having peak or maximum pressures in the range of 5 MPa to 20 MPa. In some embodiments, the photoacoustic catheters herein can generate acoustic pressure waves having peak pressures of about 1 MPa, 2 MPa, 3 MPa, 4 MPa, 5 MPa, 6 MPa, 7 MPa, 8 MPa, 9 MPa, 10 MPa, 11 MPa, 12 MPa, 13 MPa, 14 MPa, 15 MPa, 16 MPa, 17 MPa, 18 MPa, 19 MPa, 20 MPa, 21 MPa, 22 MPa, 23 MPa, 24 MPa, or 25 MPa. It will be appreciated that photoacoustic catheters herein can generate acoustic pressure waves having operating pressures or peak pressures that can fall within a range, wherein any of the forgoing numbers can serve as the lower or upper bound of the range, provided that the lower bound of the range is a value less than the upper bound of the range.

Therapeutic treatment can act via a fatigue mechanism or a brute force mechanism. For a fatigue mechanism, operating pressures would be about 0.5 MPa to 2 MPa, or about 1 MPa. For a brute force mechanism, operating pressures would be about 20 MPa to 30 MPa, or about 25 MPa. Pressures between the extreme ends of these two ranges may act upon a calcified lesion using a combination of a fatigue mechanism and a brute force mechanism.

It should be noted that, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

All publications and patent applications in this specification are indicative of the level of ordinary skill in the art to which this invention pertains.

As used herein, the recitation of numerical ranges by endpoints shall include all numbers subsumed within that range (e.g., 2 to 8 includes 2.1, 2.8, 5.3, 7, etc.).

The embodiments described herein are not intended to be exhaustive or to limit the invention to the precise forms disclosed in the following detailed description. Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the scope of the disclosure and within the scope of the invention defined by the appended claims.

## Claims

1. A photoacoustic catheter (2000) adapted for placement within a blood vessel having a vessel wall, the photoacoustic catheter comprising:
an elongate shaft (102) extending between a proximal region (104) and a distal region (106), the elongate shaft comprising a light guide (110) that is in optical communication with a light source (116);
a balloon (2002) coupled to the elongate shaft, the balloon selectively expanding from a collapsed configuration suitable for advancing the photoacoustic catheter through a patient's vasculature to a first expanded configuration suitable for anchoring the balloon in position relative to a treatment site;
wherein the balloon is an outer balloon (2002), the photoacoustic catheter further comprising a the- second, inner balloon (2004) coupled to the elongate shaft within the outer balloon, the inner balloon being configured to expand from a collapsed configuration suitable for advancing the photoacoustic catheter through a patient's vasculature to a first expanded configuration, the inner balloon comprising a photoacoustic transducer (118) disposed on a surface of the inner balloon; the photoacoustic transducer (118) being in optical communication with the light guide, the photoacoustic transducer comprising a light-absorbing material and a thermal expansion material; the outer balloon being configured to expand using an outer balloon inflation fluid (2006), and the inner balloon being configured to expand using an inner balloon inflation fluid (2008).

2. The photoacoustic catheter (2000) of claim 1, wherein the photoacoustic transducer (118) is located on one of: (i) an outer surface of the inner balloon (2004), and (ii) an inner surface of the inner balloon (2004).

3. The photoacoustic catheter (2000) of claim 1, wherein the photoacoustic transducer (118) comprises a conformal coating on a surface of the inner balloon (2004), the conformal coating extending continuously between a proximal location and a distal location on the surface of the inner balloon (2004), the conformal coating extending continuously around a circumference of the inner balloon.

4. The photoacoustic catheter (2000) of claim 1, wherein the photoacoustic transducer (118) is configured as a plurality of at least one of: (i) circumferential bars, (ii) longitudinal bars, (iii) diagonal bars, and (iv) islands.

5. The photoacoustic catheter (2000) of claim 1, wherein a distal portion of the light guide (110) includes a diffraction grating pattern configured to direct light from the light guide to one or more light pattern locations when the balloons (2002, 2004) are in the first expanded configurations.

6. The photoacoustic catheter (2000) of claim 1, wherein the balloon (2002) is configured to change between the first expanded configuration and a second, further expanded configuration.

7. The photoacoustic catheter (2000) of claim 1, wherein the thermal expansion material of the photoacoustic transducer (118) includes a polymer that is in thermal contact with the light absorbing material.

8. The photoacoustic catheter (2000) of claim 1, wherein the outer balloon inflation fluid (2006) is a liquid and the inner balloon inflation fluid (2008) is a gas.

9. The photoacoustic catheter (2000) of claim 1, wherein the inner balloon includes (2004) a concave portion when in the inner balloon is in the first expanded configuration, the photoacoustic transducer (118) being located on an outer surface of the inner balloon, and the concave portion being configured to focus at least one acoustic pressure wave from the photoacoustic transducer.

10. The photoacoustic catheter (2000) of claim 1 wherein the thermal expansion material is selected from a group consisting of polydimethylsiloxane (PDMS), polytetrafluoroethylene (PTFE), polyimide, polyisobutylene (PIB), PIB polyurethane, polyurethanes, styrene isoprene butadiene, ethylene propylene polyacrylic, ethylene acrylic, fluorosilicone, polybutadiene, polyisoprene, and thermoplastic elastomers, and the light-absorbing material is selected from a group consisting of nanoparticles, carbon nanotubes, candle soot, candle soot nanoparticles, carbon black, a nanotube array, multiwall carbon nanotubes, and light absorbing dye.

11. The photoacoustic catheter (2000) of claim 1 wherein the thermal expansion material and the light-absorbing material are positioned adjacent to one another in layers.

12. The photoacoustic catheter (2000) of claim 1 wherein the light guide (110) is an optical fiber and the light source (116) is a laser.

13. The photoacoustic catheter (2000) of claim 1, wherein a wall of the balloon (2002) comprises integrated fluid bubbles.

14. The photoacoustic catheter (2000) of claim 1, wherein the inner balloon inflation fluid (2008) is different than the outer balloon inflation fluid (2006).

## Patentansprüche

1. Fotoakustischer Katheter (2000), der angepasst ist, um in einem Blutgefäß, das eine Gefäßwand hat, angeordnet zu werden, wobei der fotoakustische Katheter aufweist:
einen länglichen Schaft (102), der sich zwischen einem proximalen Bereich (104) und einem distalen Bereich (106) erstreckt, wobei der längliche Schaft einen Lichtleiter (110) aufweist, der in optischer Kommunikation mit einer Lichtquelle (116) ist,
einen Ballon (2002), der mit dem länglichen Schaft gekoppelt ist, wobei der Ballon aus einer kollabierten Konfiguration, die zum Vorschieben des fotoakustischen Katheters durch ein Gefäßsystem eines Patienten geeignet ist, in eine erste expandierte Konfiguration, die zur Verankerung des Ballons in Position relativ zu einem Behandlungsort geeignet ist, selektiv expandierbar ist,
wobei der Ballon ein Außenballon (2002) ist, der fotoakustische Katheter ferner einen zweiten, mit dem länglichen Schaft gekoppelten Innenballon (2004) innerhalb des Außenbal-Ions aufweist, wobei der Innenballon konfiguriert ist, um sich aus einer kollabierten Konfiguration, die zum Vorschieben des fotoakustischen Katheters durch ein Gefäßsystem eines Patienten geeignet ist, in eine erste expandierte Konfiguration zu expandieren, wobei der Innenballon einen an einer Oberfläche des Innenballons angeordneten fotoakustischen Wandler (118) aufweist, wobei der fotoakustische Wandler (118) in optischer Kommunikation mit dem Lichtleiter ist, der fotoakustische Wandler ein lichtabsorbierendes Material und ein Wärmeausdehnungsmaterial aufweist, wobei der Außenballon konfiguriert ist, um unter Verwendung eines Außenballon-Aufblähungsfluids zu expandieren, und der Innenballon konfiguriert ist, um unter Verwendung eines Innenballon-Aufblähungsfluids (2008) zu expandieren.

2. Fotoakustischer Katheter (2000) nach Anspruch 1, wobei der fotoakustische Wandler (118) entweder (i) an einer Außenfläche des Innenballons (2004) oder (ii) an einer Innenfläche des Innenballons (2004) angeordnet ist.

3. Fotoakustischer Katheter (2000) nach Anspruch 1, wobei der fotoakustische Wandler (118) eine konforme Beschichtung an einer Oberfläche des Innenballons (2004) aufweist, wobei die konforme Beschichtung sich kontinuierlich zwischen einem proximalen Ort und einem distalen Ort an der Oberfläche des Innenballons (2004) erstreckt, die konforme Beschichtung sich kontinuierlich um einen Umfang des Innenballons erstreckt.

4. Fotoakustischer Katheter (2000) nach Anspruch 1, wobei der fotoakustische Wandler (118) als eine Vielzahl von (i) Umfangsstreifen und/oder (ii) Längsstreifen und/oder (iii) Diagonalstreifen und/oder (iv) Inseln konfiguriert ist.

5. Fotoakustischer Katheter (2000) nach Anspruch 1, wobei ein distaler Teil des Lichtleiters (110) ein Beugungsgittermuster aufweist, das konfiguriert ist, um Licht aus dem Lichtleiter zu einem oder mehreren Lichtmusterorten zu lenken, wenn die Ballone (2002, 2004) in den ersten expandierten Konfigurationen sind.

6. Fotoakustischer Katheter (2000) nach Anspruch 1, wobei der Ballon (2002) konfiguriert ist, um zwischen der ersten expandierten Konfiguration und einer zweiten, weiter expandierten Konfiguration zu wechseln.

7. Fotoakustischer Katheter (2000) nach Anspruch 1, wobei das Wärmeausdehnungsmaterial des fotoakustischen Wandlers (118) ein Polymer aufweist, das in thermischem Kontakt mit dem lichtabsorbierenden Material ist.

8. Fotoakustischer Katheter (2000) nach Anspruch 1, wobei das Außenballon-Aufblähungsfluid (2006) eine Flüssigkeit ist und das Innenballon-Aufblähungsfluid (2008) ein Gas ist.

9. Fotoakustischer Katheter (2000) nach Anspruch 1, wobei der Innenballon (2004) einen konkaven Bereich aufweist, wenn der Innenballon in der ersten expandierten Konfiguration ist, wobei der fotoakustische Wandler (118) an einer Außenfläche des Innenballons angeordnet ist und der konkave Bereich konfiguriert ist, um mindestens eine akustische Druckwelle aus dem fotoakustischen Wandler zu fokussieren.

10. Fotoakustischer Katheter (2000) nach Anspruch 1, wobei das Wärmeausdehnungsmaterial aus einer Gruppe ausgewählt ist, die besteht aus: Polydimethylsiloxan (PDMS), Polytetrafluorethylen (PTFE), Polyimid, Polyisobutylen (PIB), PIB Polyurethan, Polyurethane, Styren-isopren-butadien, Ethylen-propylen-polyacryl, Ethylenacryl, Fluorsilikon, Polybutadien, Polyisopren und thermoplastische Elastomere, und das lichtabsorbierende Material aus einer Gruppe ausgewählt ist, die besteht aus: Nanopartikel, Kohlenstoff-Nanoröhrchen, Kerzenruß, Kerzenruß-Nanopartikel, Ruß, ein Nanoröhrchen-Array, mehrwandige Kohlenstoff-Nanoröhrchen und lichtabsorbierender Farbstoff.

11. Fotoakustischer Katheter (2000) nach Anspruch 1, wobei das Wärmeausdehnungsmaterial und das lichtabsorbierende Material aneinander angrenzend in Schichten angeordnet sind.

12. Fotoakustischer Katheter (2000) nach Anspruch 1, wobei der Lichtleiter (110) eine optische Faser ist und die Lichtquelle (116) ein Laser ist.

13. Fotoakustischer Katheter (2000) nach Anspruch 1, wobei eine Wand des Ballons (2002) integrierte Fluidblasen aufweist.

14. Fotoakustischer Katheter (2000) nach Anspruch 1, wobei das Innenballon-Aufblähungsfluid (2008) sich von dem Außenballon-Aufblähungsfluid (2006) unterscheidet.

## Revendications

1. Cathéter photoacoustique (2000) adapté pour être placé dans un vaisseau sanguin ayant une paroi de vaisseau, le cathéter photoacoustique comprenant :
un arbre allongé (102) s'étendant entre une région proximale (104) et une région distale (106), l'arbre allongé comprenant un guide de lumière (110) qui est en communication optique avec une source de lumière (116) ;
un ballonnet (2002) couplé à l'arbre allongé, le ballonnet subissant sélectivement une expansion d'une configuration repliée appropriée pour faire avancer le cathéter photoacoustique à travers le système vasculaire d'un patient à une première configuration expansée appropriée pour ancrer le ballonnet en position par rapport à un site de traitement ;
dans lequel le ballonnet est un ballonnet externe (2002), le cathéter photoacoustique comprenant en outre un deuxième ballonnet interne (2004) couplé à l'arbre allongé à l'intérieur du ballonnet externe, le ballonnet interne étant configuré pour subir une expansion d'une configuration pliée appropriée pour faire avancer le cathéter photoacoustique à travers le système vasculaire d'un patient à une première configuration expansée, le ballonnet interne comprenant un transducteur photoacoustique (118) disposé sur une surface du ballonnet interne ; le transducteur photoacoustique (118) étant en communication optique avec le guide de lumière, le transducteur photoacoustique comprenant un matériau à absorption de lumière et un matériau à expansion thermique ;
le ballonnet externe étant configuré pour subir une expansion en utilisant un fluide de gonflage de ballonnet externe (2006), et le ballonnet interne étant configuré pour subir une expansion en utilisant un fluide de gonflage de ballonnet interne (2008).

2. Cathéter photoacoustique (2000) selon la revendication 1, dans lequel le transducteur photoacoustique (118) est positionné dans l'une parmi : (i) une surface externe du ballonnet interne (2004) et (ii) une surface interne du ballonnet interne (2004).

3. Cathéter photoacoustique (2000) selon la revendication 1, dans lequel le transducteur photoacoustique (118) comprend un revêtement conforme sur une surface du ballonnet interne (2004), le revêtement conforme s'étendant de manière continue entre un emplacement proximal et un emplacement distal sur la surface du ballonnet interne (2004), le revêtement conforme s'étendant de manière continue autour d'une circonférence du ballonnet interne.

4. Cathéter photoacoustique (2000) selon la revendication 1, dans lequel le transducteur photoacoustique (118) est configuré sous la forme d'une pluralité d'au moins l'un parmi : (i) des barres circonférentielles, (ii) des barres longitudinales, (iii) des barres diagonales et (iv) des îlots.

5. Cathéter photoacoustique (2000) selon la revendication 1, dans lequel une partie distale du guide de lumière (110) comprend un motif de réseau de diffraction configuré pour diriger la lumière du guide de lumière à un ou plusieurs emplacements de motif de lumière lorsque les ballonnets (2002, 2004) sont dans les premières configurations expansées.

6. Cathéter photoacoustique (2000) selon la revendication 1, dans lequel le ballonnet (2002) est configuré pour changer entre la première configuration expansée et une deuxième configuration plus expansée.

7. Cathéter photoacoustique (2000) selon la revendication 1, dans lequel le matériau à expansion thermique du transducteur photoacoustique (118) comprend un polymère qui est en contact thermique avec le matériau à absorption de lumière.

8. Cathéter photoacoustique (2000) selon la revendication 1, dans lequel le fluide de gonflage de ballonnet externe (2006) est un liquide et le fluide de gonflage de ballonnet interne (2008) est un gaz.

9. Cathéter photoacoustique (2000) selon la revendication 1, dans lequel le ballonnet interne (2004) comprend une partie concave lorsque le ballonnet interne est dans la première configuration expansée, le transducteur photoacoustique (118) étant positionné sur une surface externe du ballonnet interne, et la partie concave étant configurée pour concentrer au moins une onde de pression acoustique du transducteur photoacoustique.

10. Cathéter photoacoustique (2000) selon la revendication 1, dans lequel le matériau à expansion thermique est sélectionné dans un groupe comprenant le polydiméthylsiloxane (PDMS), le polytétrafluoroéthylène (PTFE), le polyimide, le polyisobutylène (PIB), le PIB polyuréthane, les polyuréthanes, le styrène-isoprène-butadiène, un (copolymère d')éthylène-acide acrylique, l'acrylique d'éthylène, le fluorosilicone, le polybutadiène, le polyisoprène et les élastomères thermoplastiques et le matériau à absorption de lumière est sélectionné dans un groupe comprenant des nanoparticules, des nanotubes de carbone, la suie de bougie, des nanoparticules de suie de bougie, du noir de carbone, une matrice de nanotubes, des nanotubes de carbone multiparois, et un colorant absorbant la lumière.

11. Cathéter photoacoustique (2000) selon la revendication 1, dans lequel le matériau à expansion thermique et le matériau à absorption de lumière sont positionnés de manière adjacente entre eux en couches.

12. Cathéter photoacoustique (2000) selon la revendication 1, dans lequel le guide de lumière (110) est une fibre optique et la source de lumière (116) est un laser.

13. Cathéter photoacoustique (2000) selon la revendication 1, dans lequel une paroi du ballonnet (2002) comprend des bulles de fluide intégrées.

14. Cathéter photoacoustique (2000) selon la revendication 1, dans lequel le fluide de gonflage de ballonnet interne (2008) est différent du fluide de gonflage de ballonnet externe (2006).
